(19)

**Europäisches Patentamt**
**European Patent Office**
**Office européen des brevets**

(11)  **EP 4 376 940 B1**

(12)  **EUROPEAN PATENT SPECIFICATION**

(45) Date of publication and mention
of the grant of the patent:
**09.07.2025   Bulletin 2025/28**

(21) Application number: **22754402.0**

(22) Date of filing: **22.07.2022**

(51) International Patent Classification (IPC):
*A61N 1/32* (2006.01)        *A61N 5/02* (2006.01)
*A61B 18/18* (2006.01)       *A61B 18/00* (2006.01)
*A61N 5/04* (2006.01)

(52) Cooperative Patent Classification (CPC):
**A61N 1/328; A61B 18/1815;** A61B 2018/00577;
A61N 5/04

(86) International application number:
**PCT/EP2022/070713**

(87) International publication number:
**WO 2023/006633 (02.02.2023 Gazette 2023/05)**

(54) **HANDHELD APPLICATOR AND A SKIN TREATMENT KIT**

**TRAGBARER APPLIKATOR UND HAUTBEHANDLUNGSKIT**

**APPLICATEUR PORTATIF ET KIT DE TRAITEMENT DE LA PEAU**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **29.07.2021  GB 202110955**

(43) Date of publication of application:
**05.06.2024   Bulletin 2024/23**

(73) Proprietor: **Bangor University**
**Bangor**
**Gwynedd LL57 2DG (GB)**

(72) Inventors:
• **JONES, Aeron Wyn**
**Bangor Gwynedd LL57 2DG (GB)**

• **ULLRICH, George**
**Bethesda Gwynedd LL57 4YY Wales (GB)**
• **HANCOCK, Christopher, Paul**
**Bangor Gwynedd LL57 2DG (GB)**
• **WEBB, David**
**Bethesda Gwynedd LL57 4YY Wales (GB)**

(74) Representative: **Mewburn Ellis LLP**
**Aurora Building**
**Counterslip**
**Bristol BS1 6BX (GB)**

(56) References cited:
**GB-A- 2 467 604        KR-A- 20200 131 709**
**US-A1- 2010 296 977    US-A1- 2015 025 513**
**US-B2- 10 779 885**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

<u>TECHNICAL FIELD</u>

The invention is directed to a handheld applicator for treating a skin by means of microwave radiation. The handheld applicator comprises an applicator housing and a microwave emitter configured to emit microwave radiation for treating tissue in a target area.

The invention is also directed to a skin treatment kit, comprising the handheld applicator and the applicator pad.

<u>BACKGROUND OF THE INVENTION</u>

There are various types of skin treatment systems that use ablation or heating of the skin to treat skin disease or to rejuvenate the skin. Skin treatment is a growing market, and there is a consumer-led desire for non-invasive treatments for disease and skin rejuvenation.

The skin is the largest organ of the human body and is made up of different layers each of which have a different composition which causes a different reaction to heating in each layer.

Ablation prompts a number of responses from skin tissue. For example, ablation of a region of skin tissue can stop that region from functioning and may stimulate healing in the skin as the ablated region is treated as a wound by the skin. Ablation may be used in the treatment of skin cancer, lesions, or tumours. Treatment of skin tissue with sufficient heating can stimulate sub-surface changes in the skin by denaturing proteins causing a healing response from the skin tissue. It is known that the use of heat beneath the surface of the skin, even without causing ablation, can stimulate new skin growth and collagen growth which will cause skin to look more youthful, cause the skin to tighten and reduce the severity of wrinkles.

It is known to use controlled radio frequency (RF) electromagnetic radiation, e.g. with a frequency between 10 kHz and 300 MHz, and/or infrared or near infrared laser light to cause heating in skin tissue. Skin appears as a dielectric medium to RF radiation. When RF radiation passes through the skin, heat is generated at and beneath the surface of the skin tissue. The laser light heats skin tissue by absorption of the laser light as it passes into the skin.

In combination with the application of low frequency RF electromagnetic radiation and/or laser light, it is also known to cool the surface of the skin to reduce the risk of damage or burning at the surface due to excessive heating. Cooling can, for example, be achieved through the use of cryogenic sprays or thermo-electric cooling using Freon, tetrafluoroethane or other such refrigerants.

There are a number of drawbacks of using RF electromagnetic radiation. RF electromagnetic waves penetrate deep into the skin, which means it can be difficult to predict and control the depth to which heating occurs. Moreover, the dielectric heating induced by low frequency RF radiation causes the heat to be spread by conduction through the skin tissue. Heat transfer by conduction can lead to uneven heating, and when cooling is applied to the skin it counteracts and disrupts the conduction of heat to cause a reduced heating effect or to eliminate any heating.

Laser light is absorbed only in a small region of skin tissue where the laser light is incident. This causes significant heating in those regions generating hotspots from which heat is then transferred to the surrounding skin tissue by conduction. The hotspots can cause uneven heating and the overall heating is affected in the same way by cooling as RF radiation. The depth of penetration of laser light and therefore the heating effect depends on skin composition at the location where the laser light is applied and is therefore unpredictable. This can lead to variation in results between different parts of the body and different patients.

Recently, a skin treatment apparatus using microwave radiation was proposed (see WO 2017 /202911 A1). US 2015/025513 A1 discloses a system for providing treatment feedback for a thermal treatment device which includes a thermal transmitter that is configured to apply an amount of thermal energy to a target site. The thermal energy has a selected frequency and a selected power level for penetration to a depth beneath the surface of the skin. US 10,779,885 B2 refers to systems for delivering microwave energy to skin are provided, such that a focal zone of destructive heat is generated in the upper sub-dermis, mid-dermis, and/or lower dermis. US 2010/296977 A1 discloses a sterilisation system having a controllable (e.g. adjustably modulatable) nonionising microwave radiation source for providing microwave energy for combining with a gas (e.g. an inert gas or a mixture of inert gases) to produce atmospheric low temperature plasma for sterilising biological tissue surfaces or the like. GB 2 467 604 A refers to a skin tissue measurement and/or treatment apparatus for controllably delivering electromagnetic radiation having a frequency of 10 GHz or more directly to a localised region of skin tissue 10 via a monopole antenna 30 adapted to penetrate the skin surface.

<u>SUMMARY OF THE INVENTION</u>

An objective of the invention is to provide a skin treatment apparatus and/or components thereof which allow a better treatment of a skin of a patient.

The subject is solved by the subject matter of the independent claims. The dependent claims described preferred embodiments of the invention.

A waveguide section for directing microwave radiation to a skin comprises a waveguide body including a wall section, preferably two opposing wall sections. The wall section includes a meta surface structure, optionally having the characteristics of a quasi-perfect magnetic conductor. The meta surface structure includes a non-conductive substrate and a conductive layer which forms a pattern and is arranged on the substrate. The pattern is constituted by a plurality of identical unit cells. Each unit cell comprises at least one capacitive portion constituting a part of a capacitor within the pattern, at least one inductive portion constituting an inductor or part of an inductor within the pattern, and an intermediate portion connecting the capacitive portion to the inductive portion. The dimensions of the capacitive portion and the dimensions of the inductive portion are chosen such that the resonance frequency of an electric circuit including the capacitive portion and the inductive portion corresponds to a frequency of the microwave radiation.

A waveguide for directing microwave radiation to a skin comprises a first waveguide section, a second waveguide section, and a tapered waveguide section arranged between the first waveguide section and the second waveguide section. A cross-sectional area of the first waveguide section is smaller than a cross-sectional area of the second waveguide section. An antenna for emitting the microwave radiation is arranged within the second waveguide section.

A skin treatment apparatus for treating a skin by means of microwave radiation comprises a power source and a handpiece separate from the power source and powered by the power source. The handpiece includes a microwave source for generating microwave energy and a waveguide for directing the microwave energy to the skin.

The provision of the meta surface structure allows to provide an electrical field of the microwave radiation which is more constant (e.g. uniform) along a cross-sectional view of the waveguide section. In particular, the electrical field of the microwave radiation is approximately constant (e.g. uniform) if the two opposing wall sections include the meta surface structure. In this case, the electrical field of the microwave radiation is (approximately) constant at a treatment aperture of the waveguide section in a cross-sectional view thereof such that the intensity of microwave radiation applied to the skin is (approximately) constant over its field of application. In other words, a drop of the electric field can be avoided at the wall section(s) if the meta surface structure is provided at said wall section(s).

As a consequence, a user of the skin treatment apparatus or a handheld applicator to be described later including the waveguide section does not need to take spatial variations in the intensity of the microwave radiation into account when applying the microwave radiation. This simplifies the treatment process. In addition, the application of microwave radiation is more homogeneous resulting in an equal heating or ablation of the skin and, therefore, in better treatment results.

The waveguide, preferably including the above-described waveguide section (also called first waveguide section in the following), provides the functional requirements for generating a substantially homogeneous radiation profile of the applied microwave radiation. In particular, the waveguide connects the dimensional requirements of the second waveguide section including the antenna with the requirements of the first waveguide section which preferably includes the meta surface structure. As a consequence, the waveguide facilitates the provision of a (approximately) homogeneous intensity profile of the microwave radiation.

The handpiece of the skin treatment apparatus includes both the microwave source and the waveguide which allows to reduce the length of the transmission between the microwave source and waveguide, for example by means of a coaxial cable. Thus, energy losses can be minimised. In addition, the handpiece can be manufactured as a component which can be hand-held, thus simplifying the manoeuvrability of the skin treatment apparatus.

The skin treatment apparatus and/or the handheld applicator may be used for any treatment of the skin by means of microwave radiation, for example non-invasive treatments for disease and skin rejuvenation by means of heating of predetermined layers of the skin. The heating may result in ablation of tissue in the skin. In particular, the skin treatment apparatus is configured to perform the above-described treatment applications. The skin may be the skin of a human being or of an animal such as a mammal.

The power source may provide direct current (DC) electrical energy. The power source can include a (rechargeable) battery or accumulator. Alternatively or additionally, the power source may include a transformer which transforms incoming alternating current (AC) into a DC current. To this end, the power source may be connected to an electricity grid such that the incoming AC may be the AC of the grid. The power source is preferably capable of providing sufficient electrical energy to power the electrical components arranged in the handpiece.

The handpiece and the power source may be connected to each other by means of a power cable. The power cable can be permanently or releasably fixed to the power source and/or the handpiece.

By virtue of the power cable, the power source and the handpiece are components separate from each other such that the handpiece can be moved independently of the power source. For example, during treatment of the skin, the power source rests on a surface (for example a table) while the handpiece is moved to and held over the part of the skin to be treated.

The power source may include a holding means (e.g. a dock or stand) which holds the handpiece, for example when the handpiece is not in use.

The handpiece and/or the handheld applicator may have outer dimensions configured to be hand-held by the user of the skin treatment apparatus. The power source, the handpiece, and/or the cable may include an outer surface which can be sterilised and/or can be treated in order to reduce the bacteriological and virological loads on the outer surfaces.

The microwave source may be any type of generator which is capable of generating microwave energy. Electromagnetic microwave energy is electromagnetic energy having a frequency between 300 MHz and 300 GHz (or a wavelength between 1 mm and 1 m), in particular between 500 MHz and 100 GHz. For example, the microwave source may emit microwave radiation in any one or more of the following frequency bands: 900 MHz to 1.5 GHz, 2.2 GHz to 2.45 GHz, 5.725 GHz to 5.875 GHz, 14 GHz to 15 GHz, and 24 GHz to 24.25 GHz. Spot frequencies of 2.45 GHz, 5.8 GHz, 14.5 GHz, or 24 GHz may be used. Preferably, the microwave source provides microwave energy having a frequency of 14.5 GHz.

Preferably, the microwave source has outer dimensions that allow the microwave source to be included in the handheld handpiece. For example, the microwave source includes a Gallium Nitride (GaN) microwave power transistor that can produce up to 25W of power at 14.5GHz. The transistor may be the CMPA1-D1E025F GaN High Electron Mobility Transistor (HEMT) based monolithic microwave integrated circuit on silicon carbide substrate released by Wolfspeed.

The waveguide may comprise a waveguide body that defines a waveguide cavity having an interior electrically conductive surface dimensioned to support propagation of the microwave electromagnetic energy. The waveguide body may be solely made from a conductive material or the inner (interior) surface may be coated with a conductive material of sufficient thickness (for example larger than the skin depth corresponding to the flow of electric currents induced by the microwave radiation in the conductive material).

The waveguide cavity may be considered exhibiting different sections which, for example, are constituted by the first waveguide section, the second waveguide section, and/or the tapered waveguide section. The waveguide sections may be integrally formed or can be made of different components which are attached to each other, for example by welding. In particular, the second waveguide section may be made from commercially available waveguide sections fulfilling defined standards. For example, the second waveguide section is a WR62 waveguide.

The first waveguide section, the second waveguide section, and/or the tapered waveguide section may have an inner and/or outer rectangular cross-section. However, other shapes in the cross-sectional view of the waveguide sections are possible, such as circular, oval, or square-shaped.

The cross-section extends perpendicular to the longitudinal extension of the waveguide sections, i.e. perpendicular to the propagation of the microwave radiation through the respective waveguide sections. The waveguide sections can be dimensioned to carry the microwave frequency electromagnetic energy in a fundamental mode, which interacts with the boundary condition set up by the waveguide sections such that the electromagnetic field emitted by the waveguide is in a quasi-TEM mode.

The cross-sectional area is an area parallel to the cross-section and confined by the inner surface of the respective waveguide section. Preferably, the cross-sectional area of the first waveguide section and/or the second waveguide section is constant along their axial extension (i.e. along the direction of propagation of the microwave radiation through the waveguide section).

The inner and/or outer cross-sectional area of the tapered waveguide section at least partially changes along its axial extension. In particular, the inner cross-sectional area diminishes along the extension from the second waveguide section to the first waveguide section. The outer cross-sectional area of the tapered waveguide section may be constant. The tapered waveguide section may be considered an adapter unit for reducing the cross-sectional area such that the first waveguide section can be attached to the second waveguide section.

The first waveguide section, the second waveguide section and/or the tapered waveguide section may include a maximum diameter parallel to the plane of the cross section. The maximum diameter may be the radius in case of a circular cross-section or a diagonal line in case of a rectangular or square shaped cross-section of the respective waveguide sections. In this case, the maximum diameter of the second waveguide section is greater than the maximum diameter of the first waveguide section.

In case of a rectangular first waveguide section and/or second waveguide section the cross-sectional area may be defined by the sides of the rectangle. The difference in the cross-sectional area may be constituted in that one or both sides of the rectangular are smaller with the first waveguide section compared to the respective sides of the second waveguide section.

The antenna for emitting microwave radiation can be constituted by any means which is capable of emitting microwave radiation. For example, the second waveguide section includes a SMA (SubMiniature version A) connector. The antenna may be provided in this case as an extension of the inner conductor of the SMA cable beyond the dielectric material of the SMA cable into the cavity of the second waveguide section. In another embodiment, the connector includes the antenna, for example the antenna is fixedly connected to the connector. If the SMA cable is connected to the connector, the inner conductor of the SMA cable is electrically connected to the antenna. The antenna may be fixedly positioned within the second waveguide section.

The microwave radiation emitted by the antenna is guided by the second waveguide section to the tapered waveguide section and, subsequently, to the first waveguide section. The microwave radiation may leave the waveguide at the treatment aperture arranged at the first waveguide section.

The first waveguide section preferably includes two opposing wall sections, for example two opposing sides of a rectangular or square shaped first waveguide section. In case of a circular or oval waveguide body of the first waveguide section, the two opposing wall sections may be any two sections of the waveguide body which oppose each other. The wall section, in particular the two opposing wall sections, may be identified in that, without the presence of the meta surface structure, there is a drop of the electrical field of the microwave radiation directly adjacent to the wall section(s). This means, at the wall section(s), the electric field of the microwave radiation guided through the respective microwave sections may be parallel or almost parallel to the surface of the opposing wall sections. The pattern on the wall section(s) allows the electric fields to be present at the wall section(s) where normally, without the pattern, the electric field would be zero.

The meta surface structure is provided for reducing the drop in the electric field such that a homogeneous intensity profile is present over the cross-section of the waveguide.

By meta surface structure, a material is meant which has a negative refractive index. The meta surface structure is arranged to create a quasi-perfect magnetic conductor boundary condition at the two opposing wall sections. In contrast to a perfect magnetic conductor (PMC), the quasi-perfect magnetic conductor exhibits its characteristics solely at or close to a predetermined frequency which preferably is the frequency of the microwave radiation guided through the waveguide.

The PMC material results in zero magnetic field along its surface. The quasi-perfect magnetic conductor may be used for this purpose, e.g. a lattice that does not conduct AC currents in a particular frequency band.

The meta surface structure may be manufactured as a microstrip which can include a non-conductive substrate (for example a dielectric material, such as Ceramic Thermoset Polymer Composite) and a conductive layer which has the shape of a pattern and is arranged on the substrate. The microstrip may be provided on a substrate (such as copper); i.e. the non-conductive substrate is arranged on the copper substrate. The substrate may be each of the two opposing wall sections. This means the microstrip can be manufactured as part of the first waveguide section.

The shape and configuration of the pattern of the conductive layer provides the characteristics as a quasi-perfect magnetic conductor. The shape and the configuration of the pattern refers to the two-dimensional extension of the conductive layer since, being manufactured as a microstrip in a preferred embodiment, the conductive layer includes a constant thickness along the two-dimensional extension of the pattern.

The pattern is composed of a plurality of unit cells whereby each unit cell is identical to another unit cell. The unit cells are arranged in a periodic manner over the meta surface structure. This means, the meta surface structure includes a periodicity whose period is defined by the unit cell.

In order to provide the quasi-perfect magnetic conductor capabilities, the unit cell itself and/or the interaction of the unit cells provide an electric circuit providing the desired quasi-perfect magnetic conductor characteristics. In particular, the meta surface structure is built up by a plurality of capacitive portions and inductive portions.

In detail, each unit cell comprises at least one capacitive portion which may form a capacitor with a capacitive portion of an adjacent unit cell and/or an additional capacitive portion of the same unit cell. Similarly, the inductive portion constitutes an inductor, either alone or in conjunction with another inductive portion of an adjacent unit cell and/or an additional inductive portion of the same unit cell.

The inductive portion and the capacitive portion are electrically connected to each other by the intermediate portion. The shape of the intermediate portion can be chosen such that it provides sufficient electrical connection between the capacitive portion and the inductive portion.

The capacitive portion and/or the inductive portion may be shaped as rectangles. For example, for providing the inductor capabilities, the length of the inductive portion is significantly larger than the width of the inductive portion (e.g. the inductive portion may be substantially elongate). The inductive portion may be considered a wire. The capacitive portion may have a width and a length whose dimensions are similar to each other. For example, the capacitive portion may be a square. This allows to provide sufficient volume for gathering electrons in order to provide a capacitor.

The dimensions such as the width and/or the length of the capacitive portion and/or the inductive portion are chosen that the resonance frequency of the electric circuit including the capacitive portion and inductive portion corresponds to the frequency of the microwave radiation. The dimensions may be selected to set the resonant frequency of a resonant circuit formed by the combined inductance of all the inductive portions of the pattern and the combined capacitance of all the capacitances of the pattern.

For example, by adjusting the size of the pattern and/or the unit cell (e.g. scaling the entire shape to be larger or smaller) that in turn alters the length/widths of the pattern and/or the unit cell, the capacitance and inductance of the lattice can be modified. The modification of the shape to change the inductance and capacitance changes the resonant frequency of the pattern/lattice which dictates the operational frequency. For example, the dimensions are chosen to be most optimal at 14.5GHz. By scaling the whole lattice and/or the unit cell up in size, the resonant frequency is increased and vice versa. Scaling may occur vertically and/or horizontally to the pattern and/or the unit cell evenly so that the shape remains the same but increase or decreases in overall size. The thickness of the shape may never be scaled and remain constant regardless of the pattern's final dimensions.

The resonance frequency f can be calculated based on this formula:

$$f = \frac{1}{2\pi\sqrt{LC}}$$

L and C are determined by capacitance and inductance of the pattern within the guide. By altering the size, the dimensions of the L and C sections change which then change the resonant frequency.

The dimensions may be selected by using a simulation simulating the microstrip and its interaction with the microwave radiation. As a starting point, the response of a single unit cell or the response of a single unit cell interacting with its directly adjacent unit cells to the microwave radiation may be simulated. If the dimensions of the capacitive portion and/or the inductive portion provide the desired resonance effect, the simulation may be extended to the whole pattern.

The capacitive portion may have a width and/or length between 0.2 mm and 2 mm, in particular between 0.5 mm and 1 mm. The inductive portion may have a length between 0.2 mm and 2 mm, in particular between 0.5 mm and 1 mm, and a width between 0.05 mm and 0.5 mm, in particular between 0.1 mm and 0.3 mm. The intermediate portion may have a width between 0.2 mm and 2 mm, in particular between 0.5 mm and 1 mm.

For assessing the characteristics of the meta surface structure, the phase of the electric field of the microwave radiation at the meta surface structure is of interest. A perfect electrical conductor (PEC) in this situation is usually at 180° phase shift and a perfect magnetic conductor (PMC) would be at the point where the phase has changed by 180° (i.e. to 0°). Through the resonance, it changes from 180° to -180° and on the way passes through 0°, which is the typical response of a PMC.

Unlike the PMC, the quasi-perfect magnetic conductor exhibits some losses. For example, the input power input in the first waveguide section may be 0.5 W and the output is 0.43 W which means that 0.07 W is lost in the dielectric materials and metals within the first waveguide section. Such an amount of loss is not considered to be enough to cause any unwanted damage and shows that most of the energy is propagating to the end of the first waveguide section.

The wall section, in particular the two opposing wall sections, are preferably completely covered with the meta surface structure in the axial direction and/or in a direction perpendicular to the axial direction (e.g. a width direction of the first waveguide section in case of a rectangular configuration in a cross-sectional view).

In a preferred embodiment, the unit cells are arranged in a row, wherein preferably the pattern includes multiple rows.

The arrangement of the unit cells in rows allows the manufacturing of the pattern as microstrips. As a consequence, the pattern has a height which is the dimension particular to the extension of the row. The height of the pattern is a multiple of the height of a single row. Thus, the height of opposing wall sections cannot be arbitrarily chosen but needs to be a multiple of the height of the row if the opposing wall sections are intended to be completely covered with the pattern. In order to attach the first waveguide section to the second waveguide section which may be a standard component and, thus, has a fixed height, the tapered waveguide section is provided. The tapered waveguide section allows to connect the first waveguide section having a height which is a multiple of the height of the row to the height of the second waveguide section.

In a preferred embodiment, the unit cell comprises at least two capacitive portions wherein the inductive portion is arranged between the two capacitive portions.

Due to the separation of the two capacitive portions by the inductive portion, these two capacitive portions do not form a capacitor between each other, but with other capacitive portions either from the same unit cell or from adjacent unit cells.

In a preferred embodiment, the capacitive portion is arranged in a corner of the unit cell, wherein preferably the capacitive portion is spaced apart from a border of the unit cell, wherein further preferably the given unit cell comprises four capacitive portions each of which is arranged in a different corner of that unit cell.

The border of the unit cell may be an imaginary line which separates the unit cell from an adjacent unit cell. The border of the unit cell preferably extends around the appearance of the unit cell. Thus, a pattern may be divided into identical unit cells by means of the border. As such, the border of the unit cell does not need to coincide with an edge of the capacitive portion and/or the inductive portion.

The distance or separation of the border of the unit cell to the capacitive portion allows the formation of a capacitor with a capacitive portion of an adjacent unit cell. The gap between the two capacitive portions forming a capacitor is twice the distance of the border of the unit cell to the capacitive portion in this embodiment.

In a preferred embodiment, the inductive portion extends to the border of the unit cell, wherein preferably the inductive portion of one unit cell is connected to the inductive portion of another unit cell.

In this embodiment, two unit cells are electrically connected by means of the respective inductive portions. Preferably, the inductive portions of the two unit cells are arranged in a straight line. In this case, the inductor constituted by the two inductive portions of the two unit cells may have a length which is twice the length of the respective inductive portion. Thus, connecting two inductive portions with each other not only provides an electrical connection between unit cells but allows to provide an inductor having a length greater than the width of a unit cell. This increases the range of the lengths of the inductive portions to choose from for setting the resonance frequency of the electric circuit to the frequency of the microwave radiation.

In a preferred embodiment, the unit cell is rectangular, wherein preferably the unit cell includes two axis of mirror symmetry which are perpendicular to each other, wherein further preferably the inductive portions (for example four inductive portions) extend in a direction of the axis of mirror symmetry.

In this embodiment, the border of the unit cell is a rectangle, preferably a square. The capacitive portion, the inductive portion, and the intermediate portion may be arranged within the unit cell in such a manner that the unit cell itself exhibits mirror symmetry, preferably along two axes of mirror symmetry. The axis of mirror symmetry may extend to a middle point of the rectangular unit cell which is defined as that point at which diagonals of the rectangle/square intersect.

The unit cell may include four capacitive portions, four inductive portions and one intermediate portion. Each capacitive portion may be aligned to a corner of the unit cell but spaced apart to the border of the unit cell. Each inductive portion may extend between two capacitive portions. The intermediate portion may have a shape that also exhibits two axes of mirror symmetry which are identical to the axes of mirror symmetry of the unit cell.

The intermediate portion may be connected to each one of the capacitive portions and inductive portions. The intermediate portion may have the shape of the rectangle or square having cut-out edges (or corners). The edges (or corners) of the capacitive portions may be placed in the cut-out edges (or corners) of the intermediate portion. Each inductive portion may be in contact with one of the edges of the rectangular or square-shaped intermediate portion.

The intermediate portion may also be considered as a rectangle or square (without cut-out edges or corners) wherein each corner of the intermediate portion contacts one corner of each capacitive portion. The intermediate portion further includes rectangular stripe portions which extend between two capacitive portions. Thus, each capacitive portion is in contact with two stripe portions of the intermediate portion. Each inductive portion is in contact with a respective stripe portion. The stripe portion and the rectangle or square of the intermediate portion may be a unitary component and are identified for describing the shape of the intermediate portion.

The stripe portion may have a length between 0.2 mm and 2 mm, in particular between 0.5 mm and 1 mm, and a width between 0.05 mm and 2 mm, in particular between 0.08 mm and 1.5 mm.

In a preferred embodiment, the first waveguide section is the waveguide section as described above.

The invention is not limited to this embodiment. The waveguide may include other means for providing a homogeneous intensity distribution of the microwave radiation over the cross-section of the waveguide, in particular at the first waveguide section. The waveguide may comprise a microwave shield on an interior wall, preferably adjacent the treatment aperture, the microwave shield covering or occupying part or parts (preferably the two opposing wall sections) of the interior wall of the waveguide, preferably the first waveguide section. The purpose of the microwave shield is to flatten the energy profile. The shield may be made from any one or more of: a dielectric material (e.g. a material having a higher relative permittivity than the interior of the cavity, and preferably exhibiting low loss at the frequency of the microwave energy); a perfect magnetic conductor (PMC); and a ferrite material.

The dielectric material may be PTFE, PEEK, or ceramic. The material may have a thickness equal to or greater than 4 mm, e.g. in a range from 4 to 6mm, preferably 5 mm. The PMC material results in zero magnetic field along its surface. Any suitable artificial magnetic conductor surface may be used for this purpose, e.g. a lattice that does not conduct AC currents in a particular frequency band, or a frequency selective surfaces (FSS) based on slot or printed dipoles on a dielectric slab (e.g. arrays of elements having a square or cross or spiral shape). A ferrite material can be used to achieve a similar effect by applying an external magnetic field to tune its electric field profile.

For example, the microwave shield may comprise a coating on the surface on the interior wall or an insert placed inside the waveguide to block the section of the interior from interacting with the microwave frequency electromagnetic energy and itself interact with the microwave frequency electromagnetic energy.

The microwave shield can be arranged to shape the emitted microwave electromagnetic energy as a uniform electromagnetic field across the treatment aperture. For example, the microwave shield may be made from dielectric material or the above described meta surface structure arranged to create a quasi-perfect magnetic conductor boundary condition at the two opposing sides of the treatment aperture. The waveguide can be dimensioned to carry the microwave frequency electromagnetic energy in a fundamental mode, which interacts with the boundary condition set up by the microwave shield such that the electromagnetic field emitted by the waveguide is in a quasi-TEM mode.

The above-described meta surface structure is an example of the microwave shield.

Waveguides for electromagnetic energy may have Transverse Electric (TE) or Transverse Magnetic (TM) modes. The fundamental mode of a rectangular waveguide (a waveguide with a rectangular cross-section in a plane perpendicular to the length of the waveguide) or a circular waveguide is a TE mode. The fundamental mode of a rectangular waveguide is the TE10 mode, and the fundamental mode of a circular waveguide is the TE11 mode. Other shapes of waveguide, for example a hexagonal or square waveguide, may have different fundamental modes.

In a preferred embodiment, the first waveguide section includes a treatment aperture for being placed on the skin.

The treatment aperture may extend parallel to the cross-section of the first waveguide section. The treatment aperture is provided for emitting the microwave radiation from the waveguide (in particular from the first waveguide section) onto the skin. Thus, for treating the skin, the waveguide is placed with the treatment aperture on the skin.

In other words, the treatment aperture may be located over the region of skin, the treatment aperture being dimensioned to emit the microwave electromagnetic energy as an electromagnetic field. To improve fit and facilitate uniform delivery of energy, the waveguide may comprise a deformable contact portion for abutting the region of skin. The deformable contact portion may comprise a deformable rim on the waveguide body around the aperture. The deformable rim may be made from any suitable biocompatible material, e.g. silicone rubber or the like. The deformable rim may be detachable from the waveguide body, e.g. to allow it to be sterilised and re-used or disposed of easily. The deformable rim may have a metallised inner surface that is electrically connected to the interior electrically conductive surface of the waveguide cavity. This can ensure that the propagation of microwave energy to the region of skin is not affected by the presence of the deformable contact portion.

The deformable contact portion may be arranged as a cover mountable in or over the treatment aperture. The cover may have a shape that matches the shape of the treatment aperture. It may be mounted in, on or onto the treatment aperture by an interference fit or a snap-fit.

The cover may be transparent to the microwave energy. In this example, it may act as a lid for containing a cooling medium present in the waveguide. The cover may stop skin tissue from entering the waveguide through the aperture, which could alter the microwave frequency electromagnetic energy distribution inside the waveguide and/or the field distribution emitted into the skin from the aperture. Having a perforation in the cover may allow the cooling medium to contact the surface of the skin tissue directly. The cover may have a plurality of perforations to allow the cooling medium to be applied to the skin in multiple locations across the aperture.

The cover may act as a separator for avoiding contact of the skin with the handpiece, in particular the waveguide. This may provide a sterile contact between the skin and the handpiece if an outer surface of the handpiece cannot be sterilized to a sufficient degree. In this case, the cover can be the sterile component for being in direct contact with the skin.

The cover may be a disposable component such that a new cover may be used for every patient. Alternatively, the cover may be a component that can be sterilised using common sterilisation protocols.

In a preferred embodiment, the waveguide of the handpiece is the waveguide as described above.

However, the invention is not limited thereto. The handpiece may include waveguides having a different structural and technical configuration.

In a preferred embodiment, the microwave source is directly connected to the waveguide.

This embodiment is possible since the microwave source as well as the waveguide are both included in the handpiece. Thus, transmission losses can be minimised as the microwave radiation can be directly transferred from the microwave source to the waveguide. In particular, a conductor of the microwave source may act as an antenna arranged within the cavity of the waveguide. The antenna may be configured as a slotted microstrip antenna.

In an alternative preferred embodiment, the handpiece further comprises a microwave transmission line which connects the microwave source to the waveguide.

The microwave transmission line may be a coaxial cable which generally includes an inner conductor and an outer conductor which are coaxially arranged and separated by a dielectric material. The dielectric material as well as the outer conductor generally have a cylindrical shape. The microwave transmission line may be flexible and can be a SMA cable.

Although the microwave transmission line is interposed between the microwave source and the waveguide, transmission losses can be reduced since the distance between the microwave source and the waveguide is small due to their arrangement within the handpiece.

In a preferred embodiment, the handpiece further includes a heat sink thermally coupled to the microwave source.

The microwave source can produce a considerable amount of heat during generation of microwave energy. In order not to overheat the microwave source and/or produce an uncomfortable warmth of the handpiece, the heat sink is provided with the microwave source. For example, the microwave source is (directly) attached to the heat sink.

The heatsink is preferably made of a thermally conductive material, such as metal. For example, aluminium is chosen as it is also lightweight.

The heatsink may also provide stability to the microwave source, in particular in case the microwave source is arranged on a circuit board or is constituted by a printed circuit board. For example, the heatsink may have an extension that corresponds with the dimensions of the microwave source such that the heatsink supports the microwave source over its complete extension.

Furthermore, the heatsink preferably made from a metal material may also provide stability and/or support for the whole handpiece. This means optional other components of the handpiece may be attached to the heatsink. For example, a housing and/or the waveguide may be attached to the heatsink.

In a preferred embodiment, the heatsink includes at least one fin, wherein preferably the handpiece further includes a fan for providing a flow of air along the at least one fin.

The heatsink may comprise a base plate and at least one fin. The microwave source, the housing, the fan and/or the waveguide may be attached to the base plate. The at least one fin is arranged on the base plate and protrudes from the base plate. The at least one fin is provided for increasing the surface area of the heatsink such that the heatsink can be more efficiently cooled by the fan.

The at least one fin may comprise a direction of extension wherein the fan is arranged to provide the flow of air along the direction of extension. To this end, the fan may be arranged at a side face of the at least one fin, for example perpendicular to the direction of extension of the at least one fin.

In a preferred embodiment, the handpiece further comprises a housing, wherein preferably the housing includes vents which are optionally arranged at opposing sides of the heat.

The housing may house all components of the handpiece. This means, all components of the handpiece are arranged within the housing. The housing may include a top shell and a bottom shell which can provide a cavity for the microwave source, the heat sink, and/or the fan. The waveguide may be covered by a waveguide cover which can be considered as a part of the housing. The waveguide cover together with the top shell and the bottom shell may completely define the outer surface of the handpiece.

In an alternative embodiment, all components except the waveguide (such as the microwave source, the heatsink, and/or the fan) are arranged within the housing. In this embodiment, the outer surface of the waveguide is exposed to the outside.

The housing may comprise one, two or more vents which provide a fluid communication between the inside of the housing and the outside of the housing. The vents are provided for exchanging air between the inside of the housing and the outside of the housing, preferably driven by the operation of the fan.

Preferably, two vents are provided wherein one vent is an inlet for drawing air from the outside of the housing into the inside of the housing. The other vent is an outlet for emitting air from the inside of the housing to the outside of the housing. For example, the fan is arranged close to the inlet vent wherein the heatsink, preferably the at least one fin, is arranged between the fan and the outlet vent. This allows a continuous flow of air from the inlet vent, via the fan along the heatsink and to the outlet vent.

In a preferred embodiment, the handpiece further comprises a control portion (e.g. controller) controlling the microwave source.

The control portion may include a microprocessor and/or an input device. The microprocessor may be provided for controlling the microwave source. The microprocessor may be arranged at the microwave source. The input device may include one or more keypads, buttons, dials and/or sliders. The input device may be arranged in or at the wall of the housing such that the input device can be accessed from the outside, for example, by a human user. The input device may include a membrane which covers the keypad, the button, the dial and/or the slider in order to provide a surface which can easily sterilised or cleaned.

The input device may include an energy delivery button for starting and terminating the generation of microwave radiation. For example, microwave radiation is generated as long as the energy delivery button is pressed. The input device may further include a keypad with which the intensity of the microwave radiation can be set. Furthermore, the keypad may be used for setting the duration of the generation of the microwave radiation.

Optionally, the handpiece may further include a display for displaying the intensity of the microwave radiation and/or other features.

At its most general, the present invention provides a treatment apparatus and/or a handheld applicator for skin treatment in which the handpiece for contacting a region of skin and/or a handheld applicator are configured to establish heating or ablation at a predetermined and preferably controllable (i.e. selectable) depth below the surface of the skin tissue. The skin treatment apparatus may be configured to provide a substantially uniform heating effect.

The treatment apparatus and/or a handheld applicator can be used for treating skin tissue below the surface of the skin. Such treatment may be applicable to various stages of skin cancer, dermatitis, hair removal or collagen shrinkage.

The treatment apparatus is preferably controllable to deliver the microwave electromagnetic energy in a manner that induces a thermal profile in the region of skin, the thermal profile having a maximum temperature that occurs at a predetermined distance beneath a surface of the region of skin.

In a simplified form, the skin is composed of three layers. The top layer is called the epidermis, the middle layer is the dermis and the bottom layer is the subcutaneous layer. Using the present invention it may be possible to controllably target energy into the dermis without causing unwanted heating elsewhere. The dermis contains many of the structural features which dictate the properties of skin such as protein fibres, e.g. collagen. Hair follicles and sweat glands are also located in the dermis. As such, for treating skin tumours which have spread to the dermis and for collagen shrinkage and hair removal it is highly desirable to penetrate heat into the dermis. When heat is applied to the dermis it is desirable to protect the epidermis from damage caused by the heat. One advantage of the microwave frequency electromagnetic energy of the present invention is the ability of to deliver heat instantaneously. The skin treatment apparatus may be capable of producing controlled, predictable, and uniform heating or ablation in the dermis.

The invention may also provide a method of treating skin tissue with microwave frequency electromagnetic energy, the method comprising: positioning the handpiece of the skin treatment apparatus described above over a region of skin; launching microwave electromagnetic energy into the waveguide; and controlling delivery of the microwave electromagnetic energy in a manner that induces a thermal profile in the region of skin, the thermal profile having a maximum temperature that occurs at a predetermined distance beneath a surface of the region of skin.

Preferably, the treatment aperture of the waveguide may have a cover, and when the aperture is positioned over the treatment region of skin tissue the cover may be in contact with the surface of the treatment region of skin tissue.

In order to shrink collagen the skin treatment apparatus preferably must be capable of producing heat between 40°C and 60°C at a depth beneath the surface of the skin tissue. In an embodiment, the heating profile provided by the device may be controllable (e.g. selectable) to target a primary heating effect (i.e. a region or layer at which maximum heating occurs) at a selectable depth below the surface. The selectable depth may be in the range of 0.2 mm to 5 mm beneath the skin surface.

The use of microwave frequency electromagnetic energy addresses problems posed by the use of laser light and/or low frequency RF electromagnetic radiation in heating skin tissue. Due to the wavelength of microwave frequency electromagnetic waves the skin effect causes the microwaves to be absorbed by the skin tissue causing controllable heating of the skin tissue over a predictable and repeatable distance and distribution. The depth of penetration allows for evenly distributed heating over a large area and to a calculable depth. Because heat is generated in the skin tissue by absorption of the microwave frequency electromagnetic energy heat is produced quicker than with low frequency RF radiation. The quicker heating effect caused by microwave frequency electromagnetic energy than the use of low frequency RF radiation may also improve the healing process.

The invention further relates to a handheld applicator for treating a skin by means of microwave radiation. The handheld applicator comprises an applicator housing, a microwave emitter, and an actuator assembly. The microwave emitter is configured to emit microwave radiation for treating tissue in a target area. The actuator assembly is configured to move the microwave emitter relative to the applicator housing whilst emitting microwave radiation for increasing uniformity of tissue treatment in the target area.

The handheld applicator may be a part of or is the handpiece as described above. The above-described remarks on the skin, the microwave radiation and/or tissue treatment equally apply.

The applicator is configured and/or shaped to be manually held by a user (e.g. a hand of the user). Thus, the applicator has a weight and dimensions which allow the user to place the handheld applicator on tissue or skin to be treated.

The applicator housing may include the characteristics, features, and/or optional embodiments of the housing as described above. However, the applicator housing may include any components, parts, and/or structures which hold and/or support the microwave emitter, the actuator assembly, and/or any other components arranged with the handheld applicator and/or the handpiece.

The microwave emitter includes any components, parts, and/or structures which relate to the emission of microwave radiation. The microwave emitter may not include a generator for generating microwave radiation. The microwave radiation may be generated by the microwave source described above which can be arranged within the handpiece/applicator. However, it is also possible that the microwave source is arranged external to the handpiece/applicator. The microwave source may be arranged in a base portion.

The base portion may include the above-described power source for providing direct current (DC) electrical energy to the handheld applicator. The base portion can include a (rechargeable) battery or accumulator. Alternatively or additionally, the base portion may include a transformer which transforms incoming alternating current (AC) into a DC current. To this end, the base portion may be connected to an electricity grid such that the incoming AC may be the AC of the grid. The base portion is preferably capable of providing sufficient electrical energy to power the electrical components arranged in the handheld applicator.

The handheld applicator and the base portion may be connected to each other by means of a power cable such as the power cable described above. The power cable can be permanently or releasably fixed to the base portion and/or the handheld applicator.

By virtue of the power cable, the base portion and the handheld applicator are components separate from each other such that the handpiece can be moved independently of the base portion. For example, during treatment of the skin, the base portion rests on a surface (for example a table) while the handheld applicator is moved to and held over the part of the skin to be treated.

The base portion may include a holding means (e.g. a dock or stand) which holds the handheld applicator, for example when the handheld applicator is not in use.

The microwave source may have the configuration and/or characteristics as described above. The microwave source may be arranged within the base portion. The microwave radiation generated by the microwave source may be supplied to the handheld applicated by use of a coaxial transmission line, such as a coaxial cable. The coaxial cable and the power cable may be bundled together. The coaxial cable may be permanently or releasably connected to the handheld applicator and/or the base portion.

The base portion, the handheld applicator, the power cable and/or the coaxial cable may include an outer surface which can be sterilised and/or can be treated in order to reduce the bacteriological and virological loads on the outer surfaces.

The microwave emitter may include a transmission line and an antenna structure. The transmission line may include a rigid or flexible (coaxial) cable that supplies microwave radiation from a connector to the antenna structure. The transmission line may include the characteristics, features and/or optional embodiments of the above-described microwave transmission line. The connector may be configured to be permanently or releasably connected to the coaxial cable which is connected to the base portion.

The antenna structure is any means for emitting microwave radiation. The emitted microwave radiation exits the handheld applicator and can be directed to the skin or tissue to be treated. The antenna structure can be the exposed inner conductor of a coaxial cable. The microwave radiation emitted by the antenna structure is used for treating tissue in a target area.

The antenna can be configured as described in conjunction with the handpiece. The antenna is configured to emit an electric field and is optionally positioned within the waveguide and spaced apart from the closed end of the waveguide. The distance of the antenna to the closed end of the waveguide may be equal to $((2n-1)*\lambda)/4$, wherein $\lambda$ is wavelength of the microwave radiation and n is an integer equal to or greater than 0.

Alternatively, the antenna is configured to emit a magnetic field. In this case, the antenna is positioned at or close to the closed end of the waveguide.

The actuator assembly can include one or more motors for moving the microwave emitter, for example the antenna structure. The movement of the microwave emitter occurs relative to the housing such that the intensity profile of the emitted microwave radiation changes in the target area without moving the handheld applicator or changing emission characteristics of the emitted radiation. Thus, the actuator assembly is used for spatially and temporally changing the microwave radiation that impinges onto or is absorbed by the tissue or skin in the target area. This spatial and temporal change in the emission profile of the microwave radiation emitted by the handheld applicator is used for increasing uniformity of tissue treatment.

As described above, conventional microwave waveguides have an uneven or non-uniform emission profile of microwave radiation. Thus, directing such an uneven or non-uniform emission profile to the target area would result in a non-uniform treatment of the tissue in the target area. By moving the microwave emitter, the overall emission profile of the handheld applicator can be smoothed (e.g. made more uniform). Spatially moving the emission profile of the intrinsically non-uniform emission profile of the microwave emitter smooths the overall emission profile and, thus, the treatment effect on the skin. The overall emission profile may be understood as the intensity of the microwave radiation at any given point of the target area after the completion of a predetermined movement pattern of the microwave emitter by the actuator assembly.

In other words, the total intensity of the microwave radiation after the completion of the movement pattern of the microwave emitter by the actuator assembly at a particular point in the target area can be more similar to the total intensity of the microwave radiation at another point of the target area compared to a situation in which the microwave emitter is not moved by the actuator assembly. This results in a more uniform treatment of the target area. Thus, moving the microwave emitter achieves similar results as configuring the waveguide or handpiece in such a way that the intrinsic emission profile of the microwave emitter is more uniform.

It is to be noted that the more uniform treatment of the target area by moving the microwave emitter does not necessarily imply an absolute uniform radiation intensity at any point of the target area. Rather, the movement of the microwave emitter results in a uniform treatment result of the target area. For example, moving the microwave emitter results in an even or uniform heating of the skin in the target area resulting in a more uniform treatment effect. In other words, the more uniform treatment of the target area achieved by moving the microwave emitter is the result of a particular movement of the microwave emitter in conjunction with the absorption properties and thermal conductivity properties of the tissue in the target area. The treatment of different types of tissue in the target area may require different moving patterns of the microwave emitter. What is more, even a non-uniform microwave distribution after completion of the movement of the microwave emitter may result in a uniform heating pattern of the tissue in the target area since the thermal conductivity properties and absorption properties of the tissue smooth the non-uniform radiation pattern and result in a uniform heating pattern.

The movement pattern is therefore chosen based on the intensity profile of the microwave radiation emitted by the microwave emitter as well as the thermal conductivity properties, and absorption properties of the tissue to be treated. The movement pattern may differ depending on the type of tissue to be treated and/or the depth of the tissue treatment.

The actuator assembly may be configured to translate and/or rotate the microwave emitter. The actuator assembly may be configured to vary the movement pattern of the microwave emitter depending on the tissue to be treated and, thus, on the absorption and thermal conductivity properties of the tissue to be treated.

The microwave emitter emits microwave radiation during movement by the actuator assembly. The handheld applicator and/or the base portion may include a controller to control the movement of the actuator assembly and the emission by the microwave emitter. The controller may be arranged within the base portion.

The controller may include safety features such as stopping the emission of microwave radiation if it is detected that the actuator assembly does no longer move the microwave emitter, for example if the microwave emitter is jammed. The controller may be configured to initially move the microwave emitter according to a test movement pattern which includes the complete or partial predetermined movement pattern and, if the initial or test movement pattern is successfully completed, start the predetermined movement pattern and simultaneously start the emission of the microwave radiation.

The movement pattern or the plurality of movement patterns may be stored in a memory of the handheld applicator and/or controller. The memory may be connected to the controller. The controller can include one or more processors and may be implemented by a miniature computer. The controller may include the characteristics, features and/or optional embodiments of the control portion described above.

The actuator assembly may include one or more motors and/or a gear mechanism which convert the movement generated by the motor into a movement of the microwave emitter. The motor may be a rotational electric motor and/or a linear motor. The gear mechanism may include one or more cogs, one or more axles and/or other mechanical devices for transmitting mechanical movement.

The motor may be a stepper motor for executing a predetermined movement. The stepper motor may be analogously controlled. The actuator assembly may further include an encoder which monitors the movement of the motor. The encoder may be coupled to the controller such that the controller knows whether or not the motor moves according to the predetermined movement pattern. Thus, the encoder helps to identify jamming or clogging of the motor and/or the gear mechanism.

In an optional embodiment, the microwave emitter includes a waveguide, wherein the actuator assembly is configured to move to waveguide. Further optionally, the waveguide is circular in a cross-sectional view of the waveguide.

The waveguide may have the features, characteristics and/or optional embodiments as described above. However, it is also possible that the waveguide is a standard waveguide, i.e. the inner surface of the waveguide does not include a meta surface structure. The antenna structure may be positioned within the waveguide. The waveguide may have an opening through which the microwave radiation is emitted by the antenna structure exits the waveguide and, thus, the housing. The opening may have the features, characteristics and/or optional embodiments of the treatment aperture described above.

The emission profile of the microwave radiation exiting the waveguide may be non-uniform. To counteract that non-uniform emission profile by the waveguide, the actuator assembly moves the waveguide in order to achieve the above-described improved treatment of the tissue in the target area.

A waveguide having a tubular structure - or in other words having a circular cross section - may have an oval intensity distribution of the emitted microwave radiation. Without moving in the tubular waveguide, the heating and, thus, treatment of the target area would not be uniform due to the oval intensity distribution of the emitted microwave radiation.

The waveguide or in more general terms the microwave emitter may define a treatment area which corresponds to the area which receives microwave radiation in case the waveguide or the microwave emitter is not moved by the actuator assembly. The movement of the waveguide or the microwave emitter increases the area that is treated or heated. The area which receives microwave radiation after completion of the movement pattern of the waveguide or the microwave emitter corresponds to the target area. The target area is therefore larger than the treatment area. The movement of the microwave emitter or the waveguide corresponds to the movement of the treatment area within the target area. The treatment area may have a non-uniform intensity distribution of the microwave radiation and, thus, results in non-uniform treatment of the skin in the treatment area. Thus, moving the waveguide and/or the microwave emitter not only increases the uniformity of the tissue treatment but can also increase the area of the tissue treatment.

In an optional embodiment, the actuator assembly is configured to rotate the microwave emitter around a rotation axis.

The actuator assembly may be configured to rotate the waveguide. The rotation axis may be coaxial to a main axis of the waveguide. For example, the main access of a tubular waveguide may be the axis of symmetry. It is also possible that the rotation axis coincides with the axis of symmetry such that the waveguide is spun around its axis of symmetry. In this case, the target area corresponds to the treatment area.

The predetermined movement pattern may include one or more complete rotations. However, it is also possible that the predetermined movement pattern includes half or quarter rotations of the waveguide or back and forth rotations of the waveguide.

In an optional embodiment, a centre point of the waveguide is offset to the rotation axis, wherein optionally, in a cross-sectional view of the applicator, the complete waveguide is offset to the central axis.

The centre point may be a point on the axis of symmetry of the waveguide. The centre point can also be a point in which diagonals of the shape in a cross-sectional view of the waveguide intersect. The centre point may also correspond to the centre of mass of the waveguide.

If the rotation axis is offset to the centre point of the waveguide, the waveguide does not only spin around itself, but performs a lateral movement. In this case, the target area is larger than the treatment area. In other words, the centre point of the waveguide performs a circular movement around the rotation axis. Thereby, radially inner portions of the waveguide are moved at a slower pace compared to radially outer portions of the waveguide such that particular parts of the target area are longer subjected to microwave radiation resulting in an increased heating of this particular part of the target area. However, as discussed above, the target area may still be uniformly heated due to the thermal conductivity properties and absorption properties of the tissue to be treated. It is possible that the waveguide is additionally rotated around its centre point or axis of symmetry.

In case that the complete waveguide is offset to the central axis in a cross-sectional view of the applicator, it is possible that certain parts of the target area are not subjected to microwave radiation. For example, the waveguide may perform a circular movement around the rotation axis such that the area of the target area that is directly subjected to microwave radiation has a ring shape. Thus, an area around the rotation axis may not receive microwave radiation. However, due to the increased heating of the more central ring-section of the circular treatment pattern, a uniform heating pattern of the target area can be achieved. The increased heating of the most central ring section of the circular treatment pattern results in a heating of the central portion around the rotation axis and, thus, in a uniform heating of the target area.

In an optional embodiment, the microwave emitter further includes a tube, wherein the waveguide is arranged offset to a centre axis of the tube, wherein optionally the actuator assembly is configured to rotate the tube around its centre axis.

The tube and the waveguide may be coaxially positioned. Optionally, the tube and the waveguide terminate at a common end plane which may coincide with an end of the actuator housing. The tube may have a circular cross-section. The centre axis may correspond to the axis of symmetry of the tube. The outer diameter of the waveguide may be smaller than the radius of the tube such that the waveguide does not intersect with the centre axis of the tube in a cross-sectional view of the tube.

The centre axis of the tube may correspond to the rotation axis. Therefore, rotating the tube around the centre axis results in a rotation of the waveguide around the centre axis or rotation axis. In other words, by rotating the tube around its centre axis, the waveguide is rotated around the rotation axis.

The tube and the waveguide may be a unitary component which both can be made from a metal or plastic material. For example, the tube and the waveguide may be machined out of a solid aluminium block. It is also possible that the tube and the waveguide may be 3D printed, e.g. using a plastic material, whereby the inner surface of the waveguide is coated with a metal layer to provide the reflection properties for the microwave radiation.

In an optional embodiment, the microwave emitter includes a rotatable junction for the coaxial transmission line, wherein optionally the rotatable junction is arranged on the centre axis.

The coaxial transmission line may be part of the microwave emitter as described above. Due to the rotation of the waveguide and, thus, of the antenna structure, the coaxial transmission line would be twisted during movement of the waveguide. To balance this, a rotatable junction is arranged in the transmission line for allowing twisting of the transmission line. The rotatable junction may include a QMA or QN connector which is the quick lock version of a SMA or N connector. The QMA or QN connector can rotate 360° after installation.

If the rotatable junction is arranged in line with the centre axis of the tube or the rotation axis, the complete rotational movement can be absorbed by the rotatable junction.

In an optional embodiment, a rotational axis of an axle of the motor coincides with the centre axis or the rotation axis. The gear mechanism may guide the movement around the rotatable junction if it is also arranged on the centre axis or rotation axis. Due to the alignment of the rotational axis of the motor with the rotation axis, offset forces or shear forces can be avoided which reduces the risk of jamming the gear mechanism or the rotation of the tube/waveguide. For example, a planetary gear mechanism is used to step around the rotatable junction. In this way, it is possible to get a balanced concentric drive. However, it is possible that the rotational axis of the motor is arranged offset to the rotation axis. This simplifies the gear mechanism.

The invention further refers to an applicator pad for a handheld applicator for treating a skin by means of microwave radiation. The applicator pad comprises a first portion and a second portion. The first portion is configured to be attached to the skin. The second portion is arranged opposite to the first portion for receiving the applicator. The second portion includes a holder configured to receive an insertion portion of the applicator for supporting the applicator.

The applicator may be the handheld applicator and/or the handpiece described above. The applicator pad may be used to support, hold, or fix the handheld applicator during emission of microwave radiation. This is beneficial since the movement of the microwave emitter for achieving an increased uniformity of tissue treatment requires a certain amount of time. In addition, an increase in the uniformity of tissue treatment is better achieved if the movement pattern of the microwave emitter is not counteracted by unintentional movement of the handheld applicator itself. Thus, the applicator pad supports the handheld applicator at a particular point during the emission of microwave radiation to the skin.

The applicator pad may be releasably attached close to or over the target area. For example, the applicator pad is attached to the skin by means of an adhesive tape. The applicator pad may include an adhesive tape. A double-sided adhesive tape may be used for attaching the applicator pad to the skin.

The applicator pad may be a disposable component which is disposed of after use. However, the applicator pad can be configured to be sterilised such that a repeated use of the applicator pad is possible.

The applicator pad may include a first side surface which is defined by the first portion. The first side surface or the first portion is that side of the applicator pad which are to be attached to the skin. The first side surface or the first portion may be flat or shaped in such a way that it aligns with the shape of the target area. Thus, different applicator pads may be used for different skin areas to be treated. The different applicator pads may differ in their first portion or first side surface while the second portion may be identical.

The second portion is opposite to the first portion and defines a second side surface which may be that surface of the applicator pad which is visible when the applicator pad is attached to the skin. The second surface may not have a flat surface but includes a holder for receiving an insertion portion of the applicator. The insertion portion of the applicator may be the distal end portion or distal end section of the applicator. The microwave radiation is emitted may be emitted from the distal end of the insertion portion and, thus, from the distal end of the handheld applicator.

The holder may be a socket or a through hole into which the insertion portion can be inserted. The inner dimensions of the holder correspond to the outer dimensions of the insertion portion such that the insertion portion is tightly received in the holder. In particular, after insertion of the insertion portion into the holder, the handheld applicator may not or only slightly move relative to the holder. For example, the applicator pad may support the handheld applicator even if a user does not hold the handheld applicator.

The applicator pad may generally have a ring shape or tubular shape. These shapes help to define the holder which may have a circular inner perimeter which aligns with a circular outer perimeter of the insertion portion. The applicator pad may be broader close to the first side surface compared to a region close to the opening of the holder. This helps to stabilise the applicator pad.

The applicator pad may include one or more tabs which are provided for increasing the first side surface and, thus, the area of contact with the skin. The adhesive tape may cover the one or more tabs. The tabs may be flexible which facilitates a better attachment of the applicator pad to the skin as well as a better removal the applicator pad from the skin. The tab may include gripping means (or gripping structures) with which the tabs can be peeled off the skin. The tabs may include a flexible plastic material.

In an optional embodiment, the holder is made from a rigid material, wherein optionally the complete applicator pad is made from a rigid material.

The rigid material may be a rigid plastic material. The holder is made from a rigid material in order to provide sufficient support for the handheld applicator. The complete applicator pad may be made from a rigid material, in particular in embodiments which do not include the tabs. In another embodiment, the applicator pad is made from a rigid material and a flexible material. For example, the applicator pad is made from a rigid material except for the tabs. In another embodiment, only the holder is made from a rigid material while the rest of the applicator pad is made from a flexible material.

In an optional embodiment, the applicator pad further comprises a cap transmissible to electromagnetic radiation in the visible spectrum and the microwave spectrum, wherein the cap is arranged at the first portion, wherein optionally the holder is shaped such that the insertion portion of the applicator can abut against the cap.

The cap may be in direct contact with the skin if the applicator pad is attached directly to the skin. The cap may be flush with the first side surface. The cap is arranged between the skin and the insertion portion of the handheld applicator during emission of microwave radiation. That is why, the cap needs to be transmissible for microwave radiation. The cap is also transmissible for visible light such that the applicator pad can be positioned with respect to the area to be treated while looking through the cap. This simplifies the positioning of the applicator pad and, thus, of the handheld applicator.

The cap may be a bottom surface of the holder such that the holder is a socket. The cap prevents contact of the insertion portion with the skin reducing the risk of infection of the skin by contamination by the insertion portion of the handheld applicator. This is especially relevant if the insertion portion of the applicator cannot be sterilised as required.

The holder is shaped such that the insertion portion can abut against the cap. This eliminates the risk that there is an air pocket between the insertion portion and the cap which may affect the propagation of microwave radiation. What is more, if the cap is tightly pressed against the skin the formation of an air pocket between the cap and the skin can be excluded.

The cap may be made from a flexible or rigid material. For example, Mylar is used as a material for the cap. The cap may have the shape of a disc, film, or foil which can be arranged at the bottom of the holder.

In an optional embodiment, the applicator pad further comprises a dressing transmissible for electromagnetic radiation in the visible spectrum and in the microwave spectrum and configured to be placed between the skin and the applicator pad.

The dressing may be a sterile pad or compress configured to be applied to a wound which may be the area of the skin which is subjected to microwave radiation by the handheld applicator and/or the handpiece. The dressing may be attached to promote healing and protect the wound from further harm. The dressing may be designed to be in direct contact with the wound and can be self-adhesive.

The dressing may include plastic films, gels, foams, hydrocolloids, alginates, hydrogels, and polysaccharide pastes, granules, and beads. They may provide different physical environments suited to wounds created by the application of microwave radiation. The dressing may facilitate absorption of exudate, regulate the moisture level surrounding the wound, and dry the wound. The dressing may provide for gas permeability and exchange, especially with regard to oxygen and water vapour. The dressing may mechanically debride the wound to remove slough. The dressing may be a pressure dressing that are commonly used to treat burns and after skin grafts. The dressing may apply pressure and prevent fluids from collecting in the tissue.

The dressing may be attached to the skin prior to the treatment of the skin by microwave radiation. The dressing may be arranged between the applicator pad and the skin. Thus, the microwave radiation emitted by the handheld applicator propagate through the dressing and is absorbed by the skin. Therefore, the dressing is made of a material and/or a plurality of materials which each are transmissible to microwave radiation. For example, the dressing is made from a flexible plastic material.

The dressing may cover an area of the skin which is larger than the target area. In particular, the dressing can be wider than the applicator pad. The dressing may cover the target area and a sufficiently wide ring around the target area. The dressing may be attached to the skin and, subsequently, the applicator pad is attached to the dressing. The configuration of the dressing as being transmissible for light in the visible spectrum simplifies the attachment of both the dressing as well as the applicator pad.

In an optional embodiment, the cap and/or the dressing include a visible marking indicating the target area.

The visible marking can be visible to a user and, therefore, interacts with radiation in the visible spectrum. The marking may be printed onto the cap and/or the dressing. The marking may include a protrusion and/or recess in the material of the cap and/or the dressing.

The marking may indicate the target area, i.e. the area which is subjected to microwave radiation. The marking may further indicate an area which is uniformly heated by the microwave radiation. For example, the target area may be marked by an outer ring or line, while the area which is uniformly heated by the microwave radiation is marked by an inner ring or line that may be coaxial to the outer ring. The marking may further indicate a centre of the target area, for example by means of a cross or crosshair.

A marking on the dressing may further indicate the correct position of the applicator pad. For example, the dressing and the cap include identical markings such that the correct position of the applicator pad on the dressing can be achieved by an overlap of the respective markings.

The invention may further refer to a skin treatment kit which comprises the handheld applicator as described above, and the applicator pad as described above.

In an optional embodiment, the applicator includes an insertion portion, wherein the insertion portion is configured to be tightly received in the holder. The above made remarks, configurations, and/or optional embodiments regarding the shape and dimensions of the insertion and the holder apply.

In an optional embodiment, the applicator includes a safety means configured to output a safety signal if the insertion portion is correctly positioned in the holder, wherein optionally the microwave emitter only emits microwave radiation while the safety signal is generated.

The safety means may be connected to the controller which can be configured to control the microwave source to stop the generation of microwave radiation if the safety signal generated by the safety means is no longer received. In other words, the controller controls the microwave source in such a way that microwave radiation is solely generated while the safety signal is generated which indicates that the handheld applicator is position in the holder at the correct position.

The correct position may be that position in which the insertion portion of the handheld applicator abuts against the cap. However, further definitions of the correct position are possible. The correct position may include that position in which the handheld applicator cannot move relative to the holder and/or the handheld applicator is aligned with the applicator pad in such a way that the emission profile of the microwave radiation corresponds to the markings on the dressing and/or the cap. In addition, the correct position may also correspond to a position of the handheld applicator in which no air pockets are formed between the insertion portion, the cap, the dressing, and/or the skin.

The safety means may include one or more detectors for detecting a position of the insertion portion or the handheld applicator in general. The safety means may be arranged on the applicator pad and/or the handheld applicator. For example, the safety means may include a mechanical switch which is actuated if the handheld applicator is arranged at the correct position. The switch may be arranged inside the housing and a mechanical transmission means (such as an elongate structure) which protrudes from the housing and is moved if the handheld applicator is positioned at the correct position.

The safety means may further include an optical sensor for detecting the presence of a component. This may be used for detecting whether the insertion portion abuts against the bottom of the holder. The safety means may further include an electrical contact which is closed if the handheld applicator is positioned at the correct position. For example, the insertion portion includes two electrodes insulated from each other while the applicator pad includes a counter electrode which electrically connect the two electrodes in case of the correct positioning of the handheld applicator.

The safety means may include a plurality of the above-described sensors and/or detectors which may be distributed along the outer perimeter of the insertion portion. This may facilitate a better detection of the correct position.

In an optional embodiment, the skin treatment apparatus includes a locking means for locking the insertion portion into the holder.

The locking means can be arranged on the holder and/or the applicator, in particular the insertion portion. The locking means may include any structure or component with which the applicator can be reliably attached to the holder. The locking means may include a snap-fit connection, screw connection, a snap-on interlock, and/or snap-in lock.

In the closed position of the interlocking means, the applicator may be in the correct position and can not be removed from the applicator pad without unlocking the interlocking means. Therefore, the interlocking means can be provided instead of or additionally to the safety means.

BRIEF DESCRIPTION OF THE DRAWINGS

For a better understanding of the invention, and to show how embodiments of the same may be carried into effect, reference will now be made, by way of example, to the accompanying drawings, in which:

Fig. 1 shows a perspective view of a skin treatment apparatus;
Fig. 2 shows different perspective view of a skin treatment apparatus shown in Fig. 1;
Fig. 3 shows a side perspective view of a handpiece of the skin treatment apparatus shown in Fig. 1;
Fig. 4 shows an exploded view of the handpiece of Fig. 3;
Fig. 5 shows a perspective view of the handpiece of Fig. 3 with parts of a housing being removed;
Fig. 6 shows another perspective view of the handpiece of Fig. 3;
Fig. 7 shows the perspective view of the handpiece of Fig. 6 with a cover over a treatment aperture being removed;
Fig. 8 shows schematic perspective drawing of a waveguide of the skin treatment apparatus of Fig. 1;
Fig. 9 shows a cross-sectional view of the drawing of Fig. 8;
Fig. 10 shows a perspective view of a meta surface structure provided with the waveguide of Fig. 9;
Fig. 11 shows another embodiment of the meta surface structure of Fig. 10;
Fig. 12 shows a schematic drawing of a unit cell of the meta surface structure of Figs. 10 and/or 11;
Fig. 13 shows a perspective view of another embodiment of a skin treatment kit which includes a handheld applicator;
Fig. 14 shows a perspective view of the handheld applicator of Fig. 13 whereby parts of a housing of the handheld applicator are drawn transparent;
Fig. 15 shows a perspective view of the handheld applicator of Fig. 14 whereby the housing is removed;
Fig. 16 shows a perspective view of the handheld applicator of Fig. 14 whereby the housing and further parts of the handheld applicator are removed;
Fig. 17 shows a perspective view of a motor, a gear mechanism, and a tube of the handheld applicator;
Fig. 18 shows a perspective view of another embodiment of a motor, a gear mechanism, and a tube of the handheld applicator;
Figs. 19a to 19c show various positions of the handheld applicator, an applicator pad, and a dressing during positioning these components on the skin;
Figs. 20a and 20b correspond to Figs. 19b and 19c, respectively, whereby the housing of the handheld applicator is transparent; and
Figs. 21a to 21d show various views of the applicator pad of Fig. 19.

DETAILED DESCRIPTION; FURTHER OPTIONS AND PREFERENCES

A skin treatment apparatus 10 includes a power source 12, a handpiece 14 and a power cable 16. The power source 12 may include one or more batteries or accumulators for providing direct current (DC) with which the handpiece 14 is powered (not shown in figures). The batteries of the power source 12 may be re-chargeable. Alternatively or additionally, the power source 12 may include a transformer which transforms incoming alternating current (AC) to DC (not shown in figures).

The electrical power provided by the power source 12 is transmitted to the handpiece 14 by means of the power cable 16. The power cable 16 may be fixedly or releasably attached to the power source 12 and/or the handpiece 14.

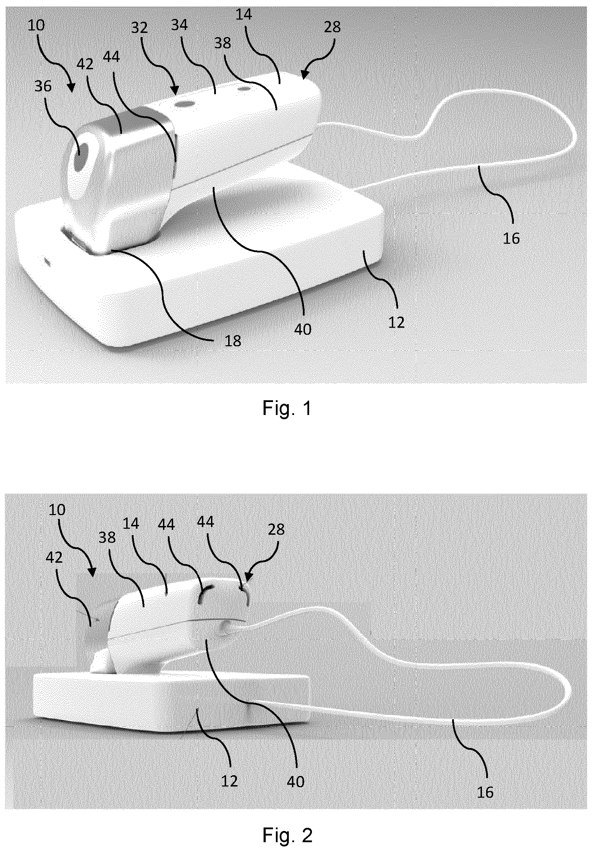

The power source 12 may include a holding means 18 for holding the handpiece 14 if the handpiece 14 is not used. In the embodiment depicted in Fig. 1, the holding means 18 is a recess in which a part of the handpiece 14 may be inserted.

The handpiece 14 generates and applies microwave radiation with which a skin can be treated. When skin tissue is irradiated with the microwave frequency electromagnetic energy, a temperature profile is established in the skin tissue. This heating temperature profile has a positive gradient towards the surface of the skin tissue, i.e. as the depth increases the temperature decreases. The heating profile is established due to absorption of the microwaves by the tissue of the skin. The skin effect dictates the absorption profile in a material, in this case skin tissue, as the distance into the material increases. The power of the microwave frequency electromagnetic energy can be manipulated to change the heating profile. The heating profile produces an ablation or heating zone at a depth below the surface of the skin tissue.

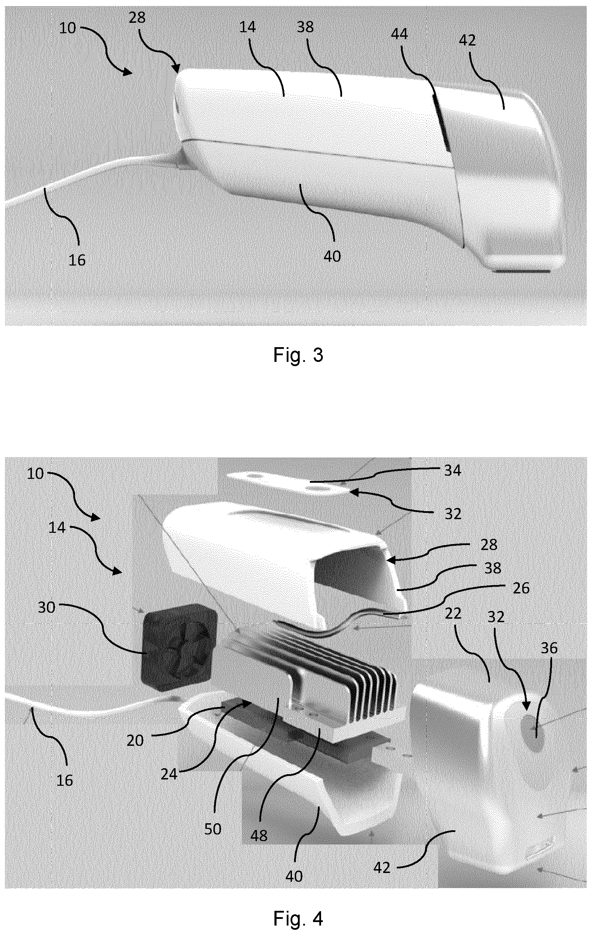

Turning to Fig. 4, the handpiece 14 may comprise a microwave source 20, a waveguide 22, a heat sink 24, a transmission line 26, a housing 28, a fan 30, and/or a control portion 32. As visible in Fig. 1, the control portion 32 may compromise an input device and a microprocessor (not shown in the figures). The input device may include a keypad 34 and an energy delivery button 36. The control portion 32 is provided for controlling the microwave source 20 i.e. is provided for controlling the generation of microwave energy and, thus, the emission of microwave radiation. Pressing the energy delivery button 36 can for example start the generation of microwave radiation while releasing the energy delivery button 36 may stop the generation of microwave radiation. The keypad 34 may include two buttons for increasing and reducing the intensity of the microwave radiation.

The control portion 32 may be electrically and/or electronically connected to the microwave source 20. The input device may be connected to the microprocessor (not shown) which may be included in the microwave source 20 or is a microprocessor separate from the microwave source 20. The microprocessor controls the microwave source 20 depending on the input by the input device. It is also possible that the microwave source 20 is directly controlled by the input device.

The control portion 32, in particular the input device, may be arranged on the housing 28 and/or in a recess of the housing 28. The energy delivery button 36 and/or the keypad 34 may be covered by a membrane which simplifies the cleaning and sterilisation of the handpiece 14.

The housing 28 provides a cover for the microwave source 20, the waveguide 22, the heat sink 24, the transmission line 26, and/or the fan 30. This means that the microwave source 20, the waveguide 22, the heat sink 24, the transmission line 26, and/or the fan 30 may be arranged within the housing 28.

The housing 28 may comprise a top shell 38, a bottom shell 40, and/or a waveguide cover 42. The top shell 38 and the bottom shell 40 may be attached to each other by means of a snap fit connection and/or adhesion. The top shell 38 and/or the bottom shell 40 may be attached to the heatsink 24. The top shell 38 and the bottom shell 40 define a cavity within which the microwave source 20, the heat sink 24, the transmission line 26, and/or the fan 30 can be arranged.

The waveguide cover 42 can be provided for covering the waveguide 22. The waveguide cover 42 may be solely attached to the waveguide 22 while the waveguide 22 is attached to the heatsink 24. Thus, the waveguide cover 42 may be not directly attached to the top shell 38 and/or the bottom shell 40.

The top shell 38, the bottom shell, and the waveguide cover 42 optionally provide a continuous surface except of the control portion 32, vents 44 and/or a treatment aperture 46. The top shell 38, the bottom shell 40, and the waveguide cover 42 may be made from a plastic material.

In the embodiment depicted in Figs. 1 to 4, the housing 28 includes four vents 44. Two vents 44 are arranged on one side of the heatsink 24 while the other two vents 44 are arranged on the other side of the heatsink 24. Thus, two vents 44 can act as an air inlet while the other two vents 44 may act as an air outlet. The arrangement of the vents 44 at both sides of the heatsink 24 allows an airflow along the heatsink 24.

The fan 30 may be provided proximate to the air inlet vents 44, in particular between the air inlet vents 44 and the heatsink 24. The far 30 is provided for generating the airflow along the heatsink 24. The fan 30 may be electrically connected to the power cable 16; the fan 30 can be powered by the power source 12.

The heatsink 24 includes a base plate 48 and a plurality of fins 50. The fins 50 are provided for increasing the surface area of the heatsink 24 such that the heatsink 24 can be better cooled by the fan 30. The heatsink 24 may be made from a metal material. The fins 50 protrude from the base plate 48 and may be integrally formed with the base plate 48. The fins 50 may be arranged parallel to each other and/or perpendicular to the fan 30. The directions of extension of the fin 50 may extend from the air inlet vents 44 to the air outlet vents 44.

The heatsink 24, in particular the base plate 48, may provide support for the microwave source 20 and/or the housing 28. The waveguide 22 may also be attached to the base plate 48.

The microwave source 20 can be considered a generator for generating microwave energy. In particular, the microwave source 20 may provide microwave energy having a frequency of 14.5 GHz. The microwave source 20 may include one or more Gallium Nitride (GaN) microwave power transistors that can produce up to 25 W of power at 14.5 GHz. The microwave power transistors may be arranged on a circuit board which in turn is directly attached to the base plate 48 in order to effectively transmit heat generated by the microwave power transistors to the heatsink 24. Generally speaking, a good transmission of thermal energy and effective cooling of the microwave source 20 is achieved if the microwave source 20 is directly attached to the heatsink 24.

In the depicted embodiment, the microwave energy is transmitted from the microwave source 20 to the waveguide 22 by means of the transmission line 26. The transmission line 26 may be a coaxial cable such as a SMA (SubMiniature version A) cable. In an alternative embodiment, not depicted in the figures, the microwave source 20 is directly attached to the waveguide 22 such that the transmission line 26 can be omitted.

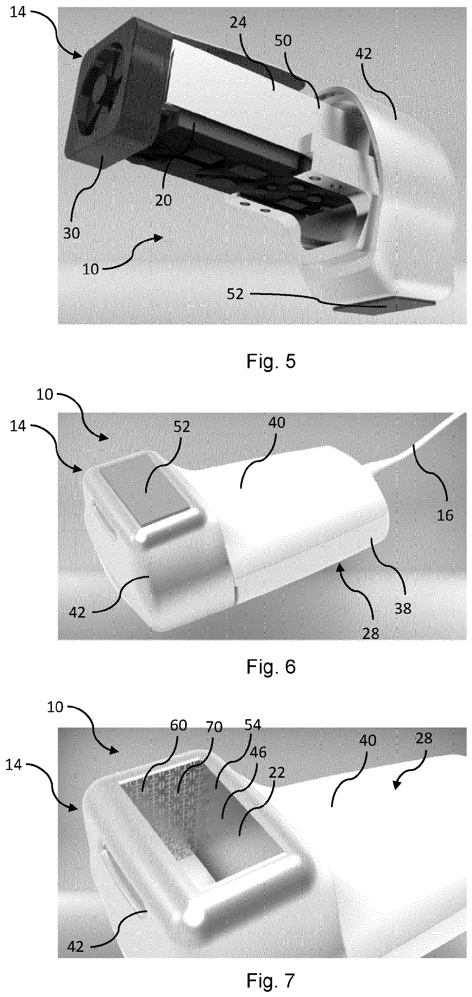

The waveguide 22 includes the treatment aperture 46 from which the microwave radiation is emitted. The treatment aperture 46 is depicted in Fig. 7 and will be discussed in the following. The treatment aperture 46 may be covered by a cover 52 which provides the contact between the skin and the handpiece 14. The cover 52 may be releasably fixed to the handpiece 14, for example by snap fitting.

The cover 52 may be a disposable component which is newly used for each person treated with the skin treatment apparatus 10. Alternatively, the cover 52 may be a component that is capable of being sterilised according to the required sterilisation protocols.

The cover 52 may be made from a material that is invisible to microwave radiation i.e. through which microwave radiation can propagate without or only minimal loss. Examples of such a material are Mylar® or a thin layer of Polytetrafluoroethylene (PTFE).

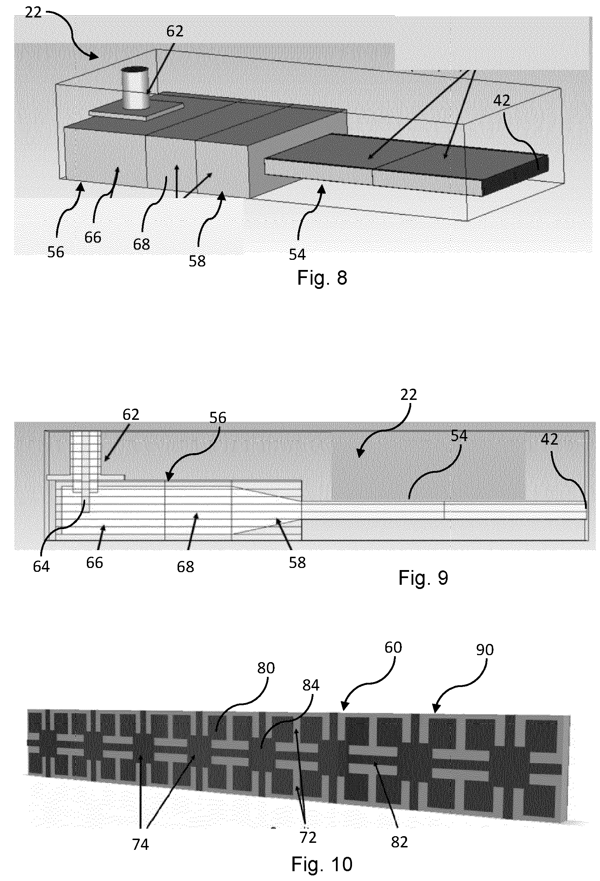

Turning to Figs. 8 and 9, the waveguide 22 includes a first waveguide section 54, a second waveguide section 56, and a tapered waveguide section 58 which each include a waveguide body. The first waveguide section 54 and the second waveguide section 56 have a constant inner cross-section along their directions of extension (corresponding to the direction of propagation of the microwave radiation) in the depicted embodiment. The first waveguide section 54 and the second waveguide section 56 may have a rectangular (e.g. inner and/or outer) cross-section. However, other cross-sectional shapes such as square-shaped, circular, or oval are possible.

A cross-sectional area (e.g. inner and/or outer) of the first waveguide section 54 is smaller than a cross-sectional area (e.g. inner and/or outer) of the second waveguide section 56. Preferably, both a width and a length of the rectangular cross-sectional area of the first waveguide section 54 are smaller than the corresponding width and length of the cross-sectional area of the second waveguide section 56.

The tapered waveguide section 58 has an inner cross-sectional area which at least partially changes along its extension from the second waveguide section 56 to the first waveguide section 54. In particular, the inner cross-sectional area of the tapered waveguide section 58 gets smaller from the second waveguide section 56 to the first waveguide section 54. Thus, the tapered waveguide section 58 acts as an adapter between the first waveguide section 54 and the second waveguide section 56. It is noted that whilst the inner cross-sectional area of the tapered waveguide section 56 tapers as described above, the outer cross-sectional area may remain constant, for example, the outer cross-sectional area may be the same as that of the second waveguide section 56 as shown in Figs. 8 and 9.

The first waveguide section 54 includes the treatment aperture 46 and a meta surface structure 60 described in the following.

The second waveguide section 56 may include a connector 62 which may be a connector for the SMA cable, for example the transmission line 26. An antenna 64 may be formed at the end of the transmission line 26 such that by attaching the transmission line 26 to the connector 62, the antenna 64 is arranged within the second waveguide section 56. The antenna 64 may be constituted by an inner conductor of the SMA cable whereby an outer conductor as well as a dielectric material acting as an insulator between the inner conductor and outer conductor is removed (see Fig. 9). However, in another embodiment, the antenna 64 may form part of the connector 62, and so the antenna 64 may be detachably coupled to the transmission line 26 and/or held in a fixed position with respect to the second waveguide section 56. In any case, the antenna 64 emits microwave radiation which is guided by the waveguide 22 to the skin. The microwave radiation leaves the waveguide 22 at the treatment aperture 46.

The second waveguide section 56 includes a regular waveguide section 66 and a matching waveguide section 68. The regular waveguide section 66 may be a commercially available waveguide such as a WR62 waveguide. The matching waveguide section 68 may have a length in the extension of the propagation of the microwave radiation which is chosen to minimise reflection of the microwave radiation at the tapered waveguide section 58.

The first waveguide section 54, the second waveguide section 56, and/or the tapered waveguide section 58 includes an inner surface which is made from an electrically conductive material, such as metal. Optionally, the waveguide body of the waveguide 22 is solely made from metal, such as copper. The waveguide 22 defines a cavity through which microwave radiation can propagate, in particular from the antenna 64 to the treatment aperture 46.

In conventional waveguides 22, the intensity of the electric field at the inner surface of the waveguide 22 depends on the orientation of the electric field with respect to the inner surface of the waveguide 22. The waveguide 22 can be dimensioned to carry the microwave frequency electromagnetic energy in a fundamental mode, which interacts with the boundary condition set up by the waveguide 22 such that the electromagnetic field emitted by the waveguide 22 is in a quasi-TEM mode. This means that the intensity of the electric field of the microwave radiation drops to zero at two opposing wall sections 70, namely if the electric field of the microwave radiation is parallel (or almost parallel) to the extension of the inner surface of the two opposing wall sections 70 of the waveguide 22. This results in an unhomogeneous intensity distribution over the cross-section of the waveguide 22. In particular, the intensity of the microwave radiation at the treatment aperture 46 is not spatially constant. This makes the treatment of the skin more complicated since the user of the skin treatment apparatus 10 needs to consider the spatially varying intensity of the microwave radiation.

In order to provide a (more) homogeneous or almost homogeneous energy distribution of the microwave radiation in the first waveguide section 54 and, in particular, at the treatment aperture 46, the meta surface structure 60 is provided at one or both of the two opposing wall sections 70. Optionally, the meta surface structure 60 is provided at the two opposing wall section 70 over the complete length of extension (which is parallel to the direction of propagation of the microwave radiation) of the first waveguide section 54. In addition, the meta surface structure 60 covers the complete width of the two opposing wall sections 70. Thus, the two opposing wall sections 70 are completely covered by the meta surface structure 60.

The two opposing wall sections 70 may be considered as extending over the complete length of the first waveguide section 54 in the axial direction thereof.

The meta surface structure 60 has the characteristics of a quasi-perfect magnetic conductor such that there is no drop of the electric field at the meta surface structure 60. As the meta surface structure 60 is not a perfect magnetic conductor, the characteristics of the perfect magnetic conductor do not appear over the whole frequency range but do appear at a predetermined frequency band which preferably corresponds to the frequency of the microwave radiation.

The meta surface structure 60 may be a microstrip having a nonconductive substrate 72 and a conductive layer 74 arranged on the nonconductive substrate 72. The conductive layer 74 forms a pattern whose shape and arrangement give rise to the quasi-perfect magnetic conductor characteristics.

The pattern and, thus, the conductive layer 74 are made up (e.g. consists) of a plurality of identical unit cells 76. This means, the plurality of unit cells 76 defines the pattern. Each unit cell 76 is defined by a (imaginary) border 78 whereby a section of the border 78 of one unit cell 76 coincides with a section of the border 78 of another unit cell 76.

Each unit cell 76 comprises at least one capacitive portion 80, at least one inductive portion 82, and at least one intermediate portion 84. The capacitive portion 80 is connected to the inductive portion 82 by the intermediate portion 84 such that the capacitive portion 80, the inductive portion 82, and the intermediate portion 84 form an electric circuit.

The capacitive portion 80 of one unit cell 76 forms a capacitor with a capacitive portion 80 of another unit cell 76. To this end, the capacitive portion 80 is spaced apart from the border 78 of the unit cell 76. Thus, there is a gap between the capacitive portion 80 of one unit cell 76 and the capacitive portion 80 of another unit cell 76. However, in an embodiment not shown in the figures, two capacitive portions 80 of one unit cell 76 form a capacitor.

The inductive portion 82 extends up to the border 78 of the unit cell 76 such that the inductive portion 82 of one unit cell 76 is connected to the inductive portion 82 of another unit cell 76. Thus, two unit cells 76 are connected by their respective inductive portions 82. Preferably, the inductive portion 82 is arranged between two capacitive portions 80 of one unit cell 76.

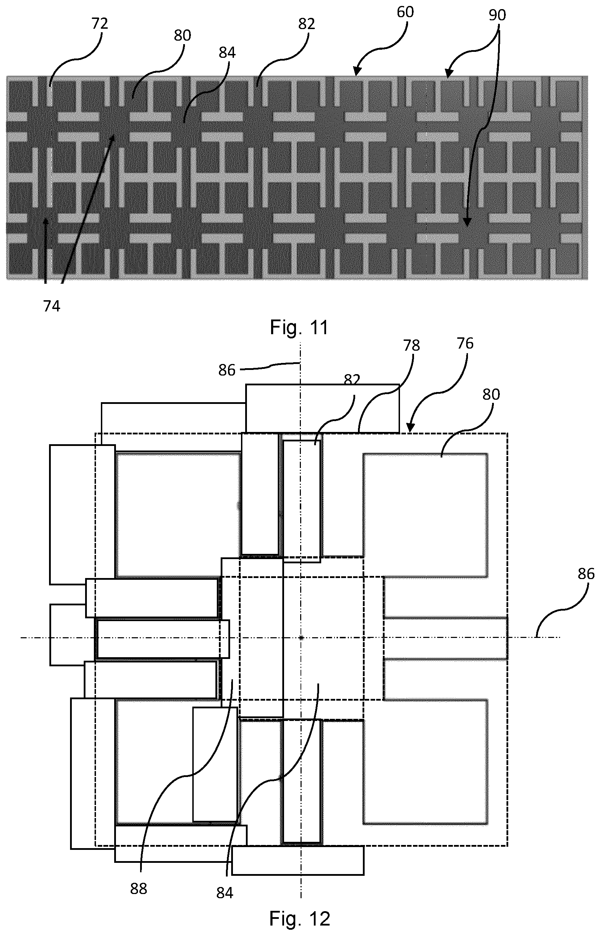

Optionally, the unit cell 76 and, in particular, the border 78 has a square shape. The unit cell 76 may include two axes of mirror symmetry 86 which are perpendicular to each other (see Fig. 12). The axes of mirror symmetry 86 extend along the direction of the inductive portions 82.

In the depicted embodiment, the capacitive portion 80 has a square shape and the inductive portion 82 has a rectangular shape. The capacitive portions 80 are aligned with the edges of the border 78. For example, four capacitive portions 80, four inductive portions 82, and one intermediate portion 84 are provided.

The intermediate portion 84 may be considered as a square wherein each corner of the intermediate portion 84 contacts one corner of each capacitive portion 80. The intermediate portion 84 further includes rectangular stripe portions 88 which extend between two capacitive portions 80. Thus, each capacitive portion 80 is in contact with two stripe portions 88 of the intermediate portion 84. Each inductive portion 82 is in contact with a single stripe portion 88. The stripe portion 88 and the rectangle of the intermediate portion 84 may be a unitary component and are identified for describing the shape of the intermediate portion 84.

The unit cells 76 are arranged in a row 90 (see Fig. 10). In order to have the possibility to increase the height of the opposing wall sections 70, a plurality of rows 90 may be provided (see Figs. 7 and 11).

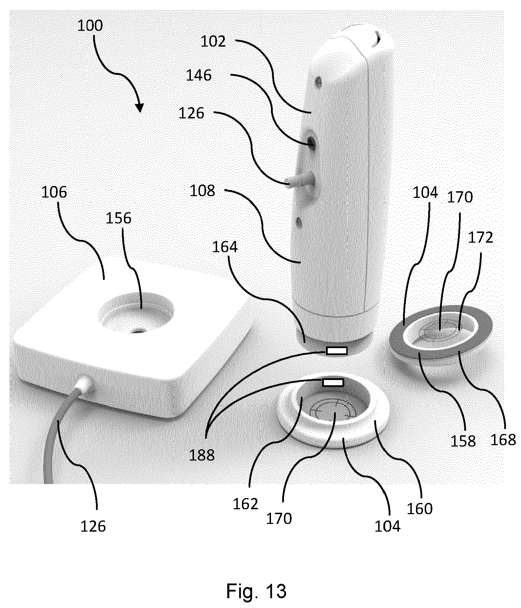

Fig. 13 shows a skin treatment kit 100 which includes a handheld applicator 102, and applicator pad 104, and a base portion 106. The handheld applicator 102 may be the handpiece 14 as described above. It is also possible that the applicator 102 has the configuration as described below.

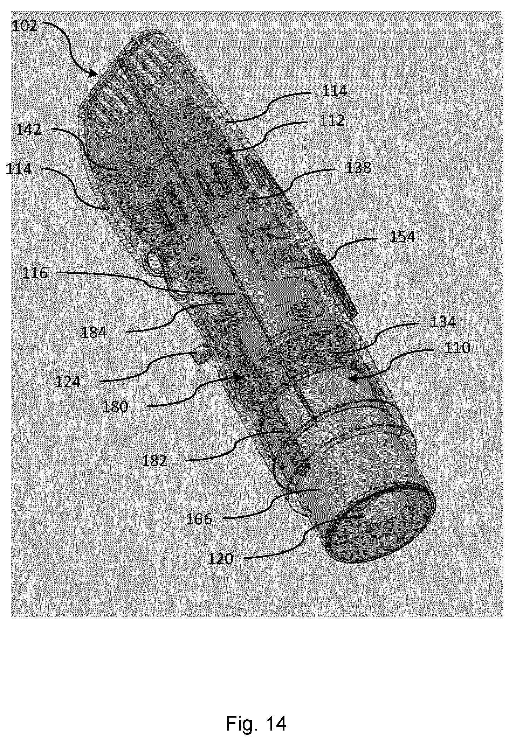

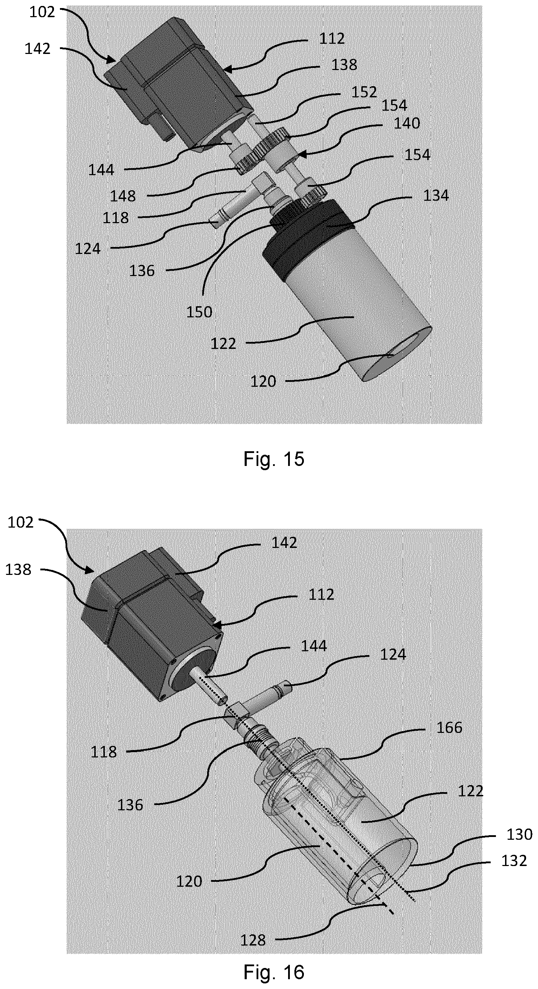

With reference to Figs. 14 to 16, the applicator 102 includes an applicator housing 108, a microwave emitter 110, and an actuator assembly 112. The applicator housing 108 is a means for supporting and shielding the microwave emitter 110, the actuator assembly 112 and/or further optional components of the handheld applicator 102. The applicator housing 108 may include one or more housing parts 114 and a support portion 116. The one or more housing parts 114 provide the outer surface of the applicator 102 similar to the housing 28 described above. The housing parts 114 may be attached to each other to form a smooth outer surface. Ventilation means such as a plurality of slits and/or through-holes may provided in the housing parts 114 for allowing air exchange between the inside of the applicator housing 108 and the surroundings of the applicator housing 108. The applicator housing 108 or the housing parts 114 may completely or partially cover the components arranged within the housing parts 114.

The housing part 114 may be attached to the support portion 116 which can be a solid structure for supporting the microwave emitter 110 and/or the actuator assembly 112. The support portion 116 may act as a support to which the microwave emitter 110, the actuator assembly 112, and/or the housing parts 114 can be attached, for example by means of screws or other fasting means.

The microwave emitter 110 may include a transmission line 118, a waveguide 120, and/or a tube 122. The transmission line 118 many include a flexible coaxial cable and/or a rigid coaxial transmission line. The transmission line 118 is configured to transmit microwave radiation from a connector 124 to the waveguide 120. The connector 124 may be configured to be permanently or releasably connected to a coaxial cable 126 which is connected to the base portion 106. The transmission line 118 is connected to an antenna (not shown in the figures) which is arranged within the waveguide 120 for emitting microwave radiation into the waveguide 120. The antenna and the part of the transmission line 118 connected to the waveguide 120 can be configured as the above-described antenna 64 and connector 62, respectively. The antenna may be spaced depart from a back surface of the waveguide 120 by a distance of $((2n-1)*\lambda)/4$, wherein $\lambda$ is wavelength of the microwave radiation and n is and integer equal to or greater than 0.

The waveguide 120 can be a circular tube defining a rotatable axis 128 which may correspond to axis of symmetry of the waveguide 120. The rotatable axis 128 extends through a centre point of the waveguide 120. The waveguide 120 has an open end at which the microwave radiation propagating within the waveguide 120 is release to the outside of the applicator 102. The open end of the waveguide 120 may correspond to a distal end 130 of the applicator 102 and can include the characteristics, features and/or optional embodiments as the treatment aperture 46 described above.

The tube 122 can be a circular tube defining a centre axis which may correspond to axis of symmetry of the tube 122. The centre axis may be a rotation axis 132 around which the waveguide 120 rotates. The centre axis or rotation axis 132 is parallel to the rotatable axis 128 and extends outside of the waveguide 120. The tube 122 is attached to the applicator housing 108, in particular the support portion 116, by means of a bearing 134 such that the tube 122 can be rotated with respect to the applicator housing 108, in particular around the rotation axis 132 or its centre axis. In other words, the actuator assembly rotates the rotatable axis 128 around the rotation axis 132. The waveguide 120 is attached to the tube 122.

The transmission line 118 may include a rotatable junction 136 which is arranged on the rotation axis 132. This allows rotation of the tube 122 and, thus, of the waveguide 120 without twisting the transmission line 118.

The actuator assembly 112 may include a motor 138, a gear mechanism 140, and an encoder 142. The motor 138 may be electric motor and have a rotation axle 144. The motor 138 may convert electrical energy into a rotational movement of the rotation axle 144. The motor 138 may be a stepper motor.

The motor 138 and the encoder 142 may be powered by electrical energy which is supplied to the applicator 102 by a power line 146 which may be permanently or releasably connected to the base portion 106 and/or the applicator 102. The motor 138 may generate sufficient rotational power for rotating the microwave emitter 110, in particular the tube 122.

The gear mechanism 140 may include a motor cog 148 attached to the rotation axle 144, a tube cog 150 attached to the tube 122, one or more transmission axles 152, and/or transmission cogs 152. The motor cog 148 and the tube cog 150 may be positioned such that they rotate around the rotation axis 132. The transmission axle 152 extends parallel to the rotation axis 132. Each end of the transmission axle 152 is provided with the transmission cogs 154 which mesh with the motor cog 148 and a tube cog 150, respectively. The gear ratio of the cogs 148, 150, and 154 may be such that the rotation of the motor 138 is downshifted.

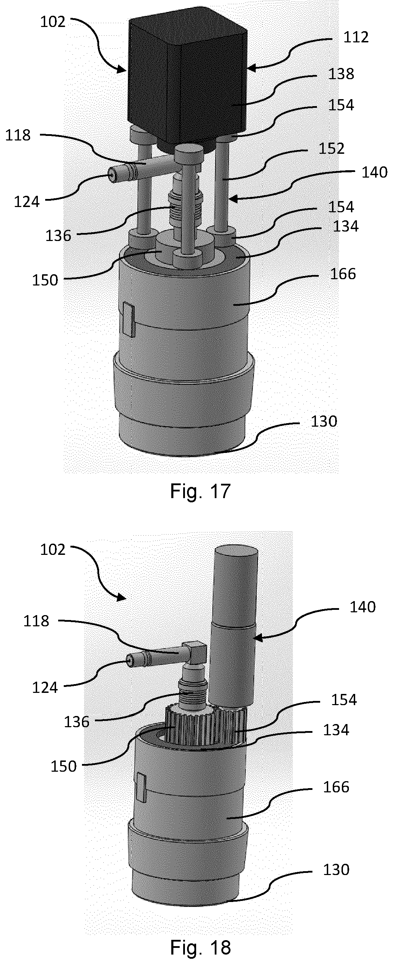

In the embodiment shown in Fig. 17, three transmission axles 152 are equally distributed around the rotation access 132. This reduces the risk of jamming of the gear mechanism 140. However, it is possible that only one transmission axle 152 is provided. It is equally possible that the rotation axle 144 is offset to the rotation axis 132 as depicted in Fig. 18. This simplifies the construction of the gear mechanism 140 since fewer cogs are required - for example, the transmission axle 152 may be directly connected to the rotation axle 144.

All the embodiments of the gear mechanism 140 described above have in common that a part of the transmission line 118 and/or the rotatable junction 136 can be positioned on the rotation axis 132 simplifying the rotation of the microwave emitter 110. However, the invention is not limited to such embodiments. For example, the actuator assembly 112 may linearly move the microwave emitter 110.

The encoder 142 is configured to monitor the rotation of the rotation axle 144. The encoder 142 may include a controller, is part of a controller, or is a separate component to the controller. The controller may also be arranged in the base portion 106. The controller controls the emission of the microwave radiation and the activation of the motor 138 based on the detection of the encoder 142. The encoder 142 monitoring the motor 138 can be essentially a binary to BCD (binary-coded decimal) counter circuit. It can include a 555 timer circuit that has two parts to generate the pulses and then goes into a counting circuit with a logic combination that stops the count once it reaches a threshold corresponding to a one or more full rotations of the motor 138.

The base portion 106 may include a power source and/or a microwave source. The power source of the base portion 106 may be the power source 12 as described above. The microwave source of the base portion 106 can be the microwave source 20 as described above. The base portion 106 may also include a stand 156 which can be configured as the holding means 18 described above. The stand 156 is configured to hold the applicator 102.

The applicator pad 104 may include a first portion 158 and second portion 160. The second portion 160 may include a holder 162 for receiving an insertion portion 164 of the applicator 102. The insertion portion 164 can coincide with the opening (the treatment aperture) of the waveguide 120. For example, the applicator housing 108 further includes a housing tube 166 which surrounds the tube 122. The housing tube 166 may be coaxially arranged to the tube 122, optionally having a small gap between the inner surface of the housing tube 166 and the outer surface of the tube 122. Optionally, the housing tube 166 may have a smaller diameter than the housing parts 114 defining an edge or stop.

Only the housing tube 166 may be configured to be inserted into the holder 162. The outer dimension and/or shape of the housing tube 166 can be adapted to be aligned to the in the dimension and/or shape of the holder 162 such that the insertion portion 164 is tightly received in the holder 162. In particular, the applicator 102 can not or only slightly move with respect to the applicator pad 104 if the applicator 102 is correctly positioned within the holder 162. In the correct position, the edge defined by the housing tube 166 and the housing parts 114 may abut against the outer edge of the holder 162. The depth of the holder 162 may correspond to the length of the housing tube 166.

The second portion 160 may define a second side surface of the applicator pad 104 which is visible when the applicator pad 104 is attached to the skin. The second portion 160 is visible in the left applicator pad 104 in Fig. 13. The right applicator pad 104 in Fig. 13 shows the first portion 158 which defines a first side surface of the applicator pad 104 to be in contact with the skin. An adhesive tape 168 can be attached to the first portion 158 for attaching the applicator pad 104 to the skin.

The applicator pad 104 may include a cap 170 which may be arranged flush with the first side surface. The cap 170 may be configured to be in direct contact with the skin. The cap 170 may be a bottom surface of the holder 162 such that the holder 162 is a socket. In the correct position, the insertion portion 164 may abut against the cap 170.

The cap 170 may include one or more markings 172. The marking 172 may indicate a target area which corresponds to that area of the skin which is subjected to microwave radiation emitted by the applicator 102. The marking 172 may also indicate an area of uniform heating which corresponds to that area within the target area which is uniformly heated by the microwave radiation. These two markings may be indicated by two rings printed on the cap 170. The marking 172 may also indicate the centre of the holder 162 which may correspond to the centre of the target area and/or the area of uniform heating. The centre of the holder 162 may be indicated by crosshair printed to the cap 170.

The cap 170 may be transmissible to light in the visible spectrum and the microwave spectrum. This allows that the cap 170 can be placed on the skin while viewing through the cap 170. In addition, microwave radiation generated by the applicator 102 can be transmitted through the cap 170.

The applicator pad 104 as depicted in Fig. 13 may have a ring-like structure and be completely made from a rigid material, such as plastic. The applicator pad 104 can be a disposable component that is disposed after use. However, the applicator pad 104 can be configured to be sterilised such that it can be repeatedly used.

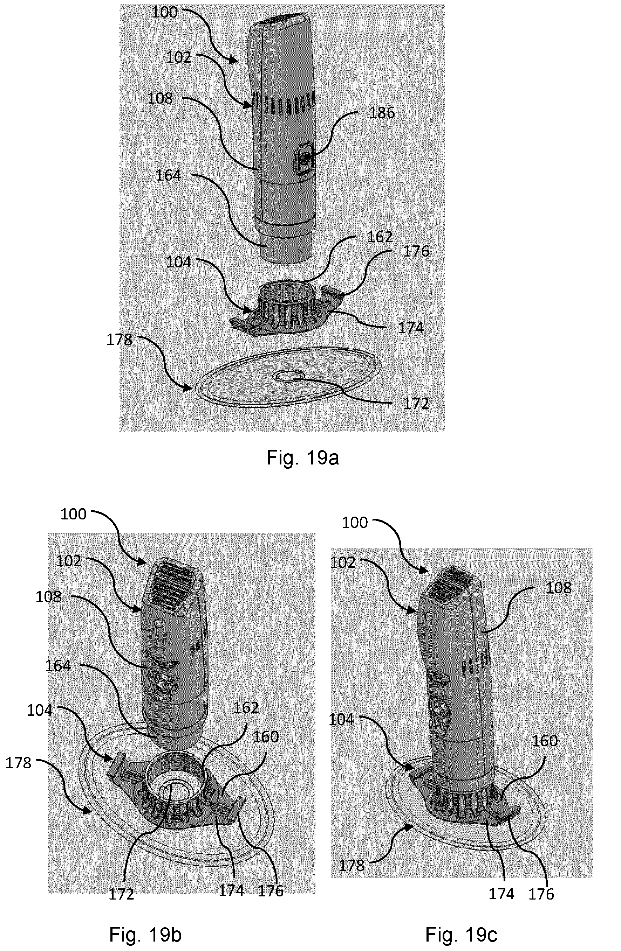

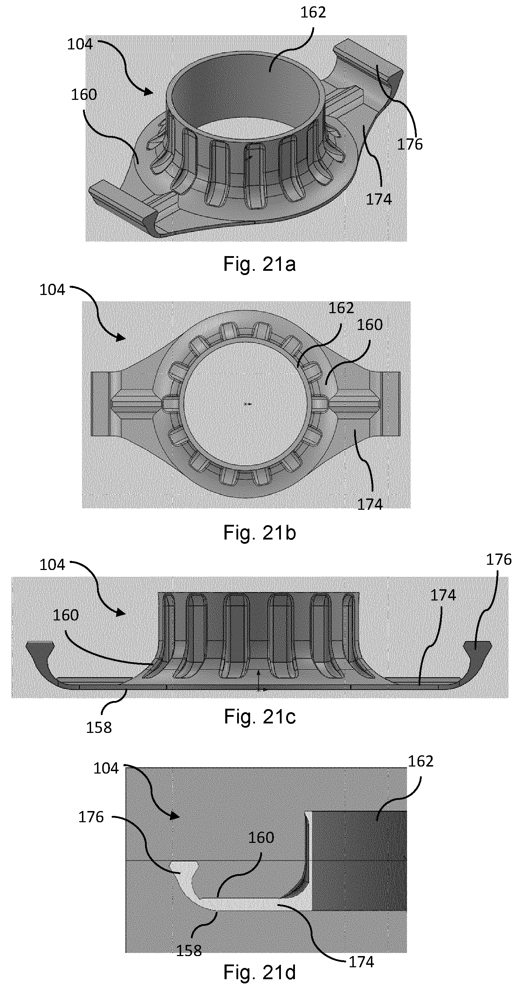

An embodiment of the applicator pad 104 as depicted in Figs. 19 to 21 is identical to the applicator pad 104 as depicted in Fig. 13 except for the following differences:

The applicator pad 104 includes one or more tabs 174 which laterally extend from the holder 162. The tabs 174 increase the area of contact with the skin and, thus, increase the first side surface of the applicator pad 104. The tabs 174 are made from a flexible material, such as a flexible plastic material, while the holder 162 is made from a rigid material for increasing to support of the applicator 102. The tabs 174 may be reinforced such that they do not break when the tabs 174 are peeled off from the skin. The first site surface of the tabs 174 may be provided with the adhesive tape 168.

The tab 174 may further include gripping means 176 which protrude from the tab 174 such that the tab 174 can be better peeled off from the skin.

The skin treatment kit 100 may further include a dressing 178. The dressing 178 may be a sterile pad or compress configured to be applied to a wound which may be the area of the skin which is subjected to microwave radiation by the handheld applicator 102. The dressing 178 may include plastic films, gels, foams, hydrocolloids, alginates, hydrogels, and polysaccharide pastes, granules, and beads.

The dressing 178 may be attached to the skin prior to the treatment of the skin by microwave radiation. The dressing 178 may be arranged between the applicator pad 104 and the skin. Thus, the microwave radiation emitted by the handheld applicator 102 propagates through the dressing 178 and is absorbed by the skin. Therefore, the dressing 178 is made of a material which is transmissible to microwave radiation.

The dressing 178 may cover an area of the skin which is larger than the target area. In particular, the dressing 178 can be wider than the applicator pad 104. The dressing 178 may be attached to the skin and, subsequently, the applicator pad 104 is attached to the dressing 178 as depicted in Figs. 19a to 19c. The dressing 178 may also include the marking 172 which can be identical to the marking 172 on the cap 170. This helps to align the applicator pad 104 to the dressing 178.

The applicator 102 may further include a safety means 180. The safety means 180 generates a safety signal if the applicator 102 is at the correct position within the holder 162. The safety means 180 is connected to the controller which controls the generation of microwave radiation only during the time the safety signal is received by the safety means 180. This facilitates that microwave radiation is solely generated if the applicator 102 is positioned in the correct position.

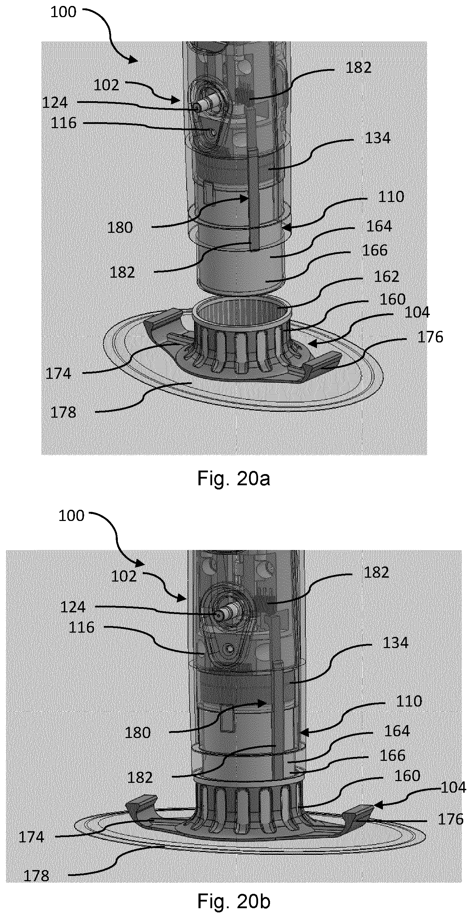

As depicted in Figs. 20a and 20b, the safety means 180 includes an elongate structure 182 and a switch 184. The elongate structure 182 protrudes from the edge defined by the housing tube 166 and the housing part 114. The elongate structure 182 is moveable within the direction of its extension. By inserting the applicator 102 into the holder 162, the elongate structure 182 is pushed into the applicator housing 108 and activates the switch 184. The elongate structure 182 and/or the switch 184 may be spring loaded towards such a position that the switch 184 is only actuated if the applicator 102 is in the correct position. The switch 184 generates the safety signal only during the time the elongate structure 182 is pushed into the applicator housing 108 by the force with which the applicator 102 is pressed into the holder 162.

The applicator 102 and/or the applicator pad 104 may also include a locking means 188. In the embodiment shown in Fig 13, the interlocking means 188 includes a component attached to the inner perimeter of the holder 162 and a component attached to the outer surface of the insertion portion 164, optionally the housing tube 166. The two components of the interlocking means 188 provide a snap-fit or snap-on connection between the applicator 102 and the applicator pad 104 for releasably attaching the applicator 102 to the applicator pad 104.

The working principle and a method for controlling the skin treatment kit 100 are described in the following:

The waveguide 120 intrinsically emits microwave radiation which has an intensity profile which is not uniform in a plane of the treatment aperture of the waveguide 120. This results in that that the skin to be treated with the microwave radiation is not uniformly heated. In order to counteract the non-uniform radiation profile of the waveguide 120 and to achieve uniform heating of the skin, the waveguide 120 is moved. In the embodiment disclosed in the figures, the waveguide 120 is rotated around the rotation axis 132, for example two times. In particular, the waveguide 120 has a movement pattern which defines a ring. The outer perimeter of this ring may correspond to the outer perimeter of the target area. An area of the target area which coincides with the middle of this ring may not be subjected to microwave radiation. However, this area is heated due to the thermal conductivity of the skin. What is more, the inventors surprisingly found that the above-described movement pattern of the waveguide 120 produces an area of uniform heating of the skin despite the fact that the target area is not uniformly subjected to microwave radiation.

The controller may start a microwave treatment routine after receiving a start signal from an input device 186 which may be a button or switch arranged on the applicator 102. The microwave treatment routine may include rotating the waveguide 120 a complete rotation around the rotation axis 132 without generating microwave radiation. This is done to check whether the gear mechanism 140 and/or the motor 138 are jammed or not. After that, the microwave treatment routine may include two complete rotations of the waveguide 120 around the rotation axis 132 during which microwave radiation is generated. The microwave treatment routine may only be started when the safety signal is received, or microwave radiation is only generated when the safety signal is received.

Although a few preferred embodiments have been shown and described, it will be appreciated by those skilled in the art that various changes and modifications might be made without departing from the scope of the invention, as defined in the appended claims.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent, or similar purpose, unless expressly stated otherwise. Thus, unless expressly stated otherwise, each feature disclosed is one example only of a generic series of equivalent or similar features.

**Claims**

1. A handheld applicator for treating a skin by means of microwave radiation, comprising

   an applicator housing (108), and
   a microwave emitter (110) configured to emit microwave radiation for treating tissue in a target area, **characterized by**
   an actuator assembly (112) configured to move the microwave emitter (110) relative to the applicator housing (108) whilst emitting microwave radiation for increasing uniformity of tissue treatment in the target area.

2. The handheld applicator according to claim 1, wherein the microwave emitter (110) includes a waveguide (120), wherein the actuator assembly (112) is configured to move the waveguide (120), wherein optionally the waveguide (120) is circular in a cross-sectional view of the waveguide (120).

3. The handheld applicator according to claim 1 or 2, wherein the actuator assembly (112) is configured to rotate the microwave emitter (110) around a rotation axis (132).

4. The handheld applicator according to claim 3, wherein, in a cross-sectional view of the applicator (102), a centre point of the waveguide (120) is offset to the rotation axis (132), wherein optionally, in a cross-sectional view of the applicator (102), the complete waveguide (120) is offset to the central axis.

5. The handheld applicator according to any one of claims 2 to 4, wherein the microwave emitter (110) further includes a tube (122), wherein the waveguide (120) is arranged offset to a centre axis of the tube (122), wherein optionally the actuator assembly (112) is configured to rotate the tube (122) around its centre axis.

6. The handheld applicator according to claim 5, wherein the microwave emitter (110) includes a rotatable junction (136) for a coaxial transmission line, wherein optionally the rotatable junction (136) is arranged on the centre axis.

7. A skin treatment kit, comprising

the handheld applicator (102) according to any one of the claims 1 to 6, and
an applicator pad (104) for the handheld applicator (102), the applicator pad (104) comprising

a first portion (158) configured to be attached to the skin,
a second portion (160) opposite to the first portion (158) for receiving the applicator (102),

wherein the second portion (160) includes a holder (162) configured to receive an insertion portion (164) of the applicator (102) for supporting the applicator (102).

8. The skin treatment kit according to claim 7, wherein the holder (162) is made from a rigid material, wherein optionally the complete applicator pad (104) is made from a rigid material.

9. The skin treatment kit according to claims 7 or 8, further comprising a cap (170) transmissible for electromagnetic radiation in the visible spectrum and the microwave spectrum, wherein the cap (170) is arranged at the first portion (158), wherein optionally the holder (162) is shaped such that the insertion portion (164) of the applicator (102) can abut against the cap (170).

10. The skin treatment kit according to any one of the claims 7 to 9, further comprising a dressing (178) transmissible for electromagnetic radiation in the visible spectrum and the microwave spectrum and configured to be placed between the skin and the applicator pad (104).

11. The skin treatment kit according to claims 9 or 10, wherein the cap (170) and/or the dressing (178) include a visible marking (172) indicating a target area.

12. The skin treatment kit according to any one of claims 7 to 11, wherein the applicator (102) includes the insertion portion (164), wherein the insertion portion (164) is configured to be tightly received in the holder (162).

13. The skin treatment kit according to any one of claims 7 to 12, further comprising a locking means (188) for locking the insertion portion (164) into the holder (162).

14. The skin treatment kit according to any one of the claims 7 to 12, wherein the applicator (102) includes a safety means (180) configured to output a safety signal if the insertion portion (164) is correctly positioned in the holder (162), wherein optionally the microwave emitter (110) only emits microwave radiation when receiving the safety signal.

**Patentansprüche**

1. Handapplikator zur Behandlung von Haut mit Mikrowellenstrahlung, der Folgendes umfasst:

ein Applikatorgehäuse (108) und
einen Mikrowellenstrahler (110), der ausgelegt ist, um Mikrowellenstrahlung für die Behandlung von Gewebe in einem Zielbereich zu emittieren, **gekennzeichnet durch**
eine Aktuatoranordnung (112), die ausgelegt ist, um den Mikrowellenstrahler (110) in Bezug auf das Applikatorgehäuse (108) zu bewegen, während Mikrowellenstrahlung emittiert wird, um die Gleichförmigkeit der Gewebebehandlung im Zielbereich zu erhöhen.

2. Handapplikator nach Anspruch 1, wobei der Mikrowellenstrahler (110) einen Wellenleiter (120) umfasst, wobei die Aktuatoranordnung (112) ausgelegt ist, um den Wellenleiter (120) zu bewegen, wobei der Wellenleiter (120) gegebenenfalls in einer Querschnittsansicht des Wellenleiters (120) kreisförmig ist.

3. Handapplikator nach Anspruch 1 oder 2, wobei die Aktuatoranordnung (112) ausgelegt ist, um den Mikrowellenstrahler (110) um eine Rotationsachse (132) zu rotieren.

4. Handapplikator nach Anspruch 3, wobei in einer Querschnittsansicht des Applikators (102) der Mittelpunkt des Wellenleiters (120) zur Rotationsachse (132) versetzt ist, wobei gegebenenfalls in einer Querschnittsansicht des Applikators (102) der komplette Wellenleiter (120) zur Mittelachse versetzt ist.

5. Handapplikator nach einem der Ansprüche 2 bis 4, wobei der Mikrowellenstrahler (110) weiters ein Rohr (122) umfasst, wobei der Wellenleiter (120) versetzt zur Mittelachse des Rohrs (122) angeordnet ist, wobei die Aktuatoranordnung (112) gegebenenfalls ausgelegt ist, um das Rohr (122) um seine Mittelachse zu rotieren.

6. Handapplikator nach Anspruch 5, wobei der Mikrowellenstrahler (110) einen rotierbaren Anschluss(136) für eine koaxiale Übertragungsleitung umfasst, wobei der rotierbare Anschluss (136) gegebenenfalls auf der Mittelachse angeordnet ist.

7. Hautbehandlungsset, das Folgendes umfasst:

einen Handapplikator (102) nach einem der Ansprüche 1 bis 6 und
eine Applikator-Auflage (104) für den Handapplikator (102), wobei die Applikator-Auflage (104) Folgendes umfasst:

einen ersten Abschnitt (158), der ausgelegt ist, um auf der Haut angebracht zu werden, einen zweiten Abschnitt (160) entgegengesetzt zum ersten Abschnitt (158) zur Aufnahme des Applikators (102),

wobei der zweite Abschnitt (160) eine Halterung (162) umfasst, die ausgelegt ist, um einen Einführungsabschnitt (164) des Applikators (102) aufzunehmen, um den Applikator (102) zu stützen.

8. Hautbehandlungsset nach Anspruch 7, wobei die Halterung (162) aus einem steifen Material besteht, wobei gegebenenfalls die gesamte Applikator-Auflage (104) aus einem steifen Material besteht.

9. Hautbehandlungsset nach Anspruch 7 oder 8, das weiters eine Kappe (170) umfasst, die für elektromagnetische Strahlung im sichtbaren Spektrum und Mikrowellenspektrum durchlässig ist, wobei die Kappe (170) am ersten Abschnitt (158) angeordnet ist, wobei die Halterung (162) gegebenenfalls so geformt ist, das der Einführungsabschnitt (164) des Applikators (102) an die Kappe (170) anstoßen kann.

10. Hautbehandlungsset nach einem der Ansprüche 7 bis 9, das weiters einen Verband (178) umfasst, der für elektromagnetische Strahlung im sichtbaren Spektrum und Mikrowellenspektrum durchlässig ist und ausgelegt ist, um zwischen der Haut und der Applikator-Auflage (104) platziert zu werden.

11. Hautbehandlungsset nach Anspruch 9 oder 10, wobei die Kappe (170) und/oder der Verband (178) eine sichtbare Markierung (172) umfassen, die einen Zielbereich anzeigt.

12. Hautbehandlungsset nach einem der Ansprüche 7 bis 11, wobei der Applikator (102) den Einführungsabschnitt (164) umfasst, wobei der Einführungsabschnitt (164) ausgelegt ist, um eng in der Halterung (162) aufgenommen zu werden.

13. Hautbehandlungsset nach einem der Ansprüche 7 bis 12, das weiters ein Verriegelungsmittel (188) umfasst, um den Einführungsabschnitt (164) in der Halterung (162) zu verriegeln.

14. Hautbehandlungsset nach einem der Ansprüche 7 bis 12, wobei der Applikator (102) ein Sicherheitsmittel (180) umfasst, das ausgelegt ist, um ein Sicherheitssignal auszugeben, wenn der Einführungsabschnitt (164) korrekt in der Halterung (162) positioniert ist, wobei der Mikrowellenstrahler (110) gegebenenfalls nur Mikrowellenstrahlung aussendet, wenn er das Sicherheitssignal empfängt.

**Revendications**

1. Applicateur portatif pour traiter une peau au moyen d'un rayonnement de micro-ondes, comprenant

un boîtier d'applicateur (108), et
un émetteur de micro-ondes (110) configuré pour émettre un rayonnement de micro-ondes afin de traiter un tissu dans une zone cible, **caractérisé par**
un ensemble actionneur (112) configuré pour déplacer l'émetteur de micro-ondes (110) par rapport au boîtier d'applicateur (108) tout en émettant un rayonnement de micro-ondes pour augmenter l'uniformité du traitement d'un tissu dans la zone cible.

2. Applicateur portatif selon la revendication 1, dans lequel l'émetteur de micro-ondes (110) inclut un guide d'ondes (120), dans lequel l'ensemble actionneur (112) est configuré pour déplacer le guide d'ondes (120), dans lequel facultativement le guide d'ondes (120) est circulaire dans une vue en coupe transversale du guide d'ondes (120).

3. Applicateur portatif selon la revendication 1 ou 2, dans lequel l'ensemble actionneur (112) est configuré pour faire tourner l'émetteur de micro-ondes (110) autour d'un axe de rotation (132).

4. Applicateur portatif selon la revendication 3, dans lequel, dans une vue en coupe transversale de l'applicateur (102), un point central du guide d'ondes (120) est décalé par rapport à l'axe de rotation (132), dans lequel facultativement, dans une vue en coupe transversale de l'applicateur (102), le guide d'ondes complet (120) est décalé par rapport à l'axe central.

5. Applicateur portatif selon l'une quelconque des revendications 2 à 4, dans lequel l'émetteur de micro-ondes (110) inclut en outre un tube (122), dans lequel le guide d'ondes (120) est agencé de manière décalée par rapport à un axe central du tube (122), dans lequel, facultativement, l'ensemble d'actionneur (112) est configuré pour faire tourner le tube (122) autour de son axe central.

6. Applicateur portatif selon la revendication 5, dans lequel l'émetteur de micro-ondes (110) inclut une jonction rotative (136) pour une ligne de transmission coaxiale, dans lequel facultativement la jonction rotative (136) est agencée sur l'axe central.

7. Kit de traitement de la peau, comprenant l'applicateur portatif (102) selon l'une quelconque des revendications 1 à 6, et

   un tampon applicateur (104) pour l'applicateur portable (102), le tampon applicateur (104) comprenant

      une première partie (158) configurée pour être fixée à la peau,
      une seconde partie (160) opposée à la première partie (158) pour recevoir l'applicateur (102),

   dans lequel la seconde partie (160) inclut un support (162) configuré pour recevoir une partie d'insertion (164) de l'applicateur (102) pour supporter l'applicateur (102).

8. Kit de traitement de la peau selon la revendication 7, dans lequel le support (162) est réalisé à partir d'un matériau rigide, dans lequel facultativement le tampon applicateur complet (104) est réalisé à partir d'un matériau rigide.

9. Kit de traitement de la peau selon la revendication 7 ou 8, comprenant en outre un capuchon (170) transmissible pour un rayonnement électromagnétique dans le spectre visible et le spectre des micro-ondes, dans lequel le capuchon (170) est agencé au niveau de la première partie (158), dans lequel facultativement le support (162) est mis en forme de telle sorte que la partie d'insertion (164) de l'applicateur (102) puisse venir en butée contre le capuchon (170).

10. Kit de traitement de la peau selon l'une quelconque des revendications 7 à 9, comprenant en outre un pansement (178) transmissible pour un rayonnement électromagnétique dans le spectre visible et le spectre des micro-ondes, et configuré pour être placé entre la peau et le tampon applicateur (104).

11. Kit de traitement de la peau selon les revendications 9 ou 10, dans lequel le capuchon (170) et/ou le pansement (178) comprennent un marquage visible (172) indiquant une zone cible.

12. Kit de traitement de la peau selon l'une quelconque des revendications 7 à 11, dans lequel l'applicateur (102) inclut la partie d'insertion (164), dans lequel la partie d'insertion (164) est configurée pour être reçue de manière serrée dans le support (162).

13. Kit de traitement de la peau selon l'une quelconque des revendications 7 à 12, comprenant en outre des moyens de verrouillage (188) pour verrouiller la partie d'insertion (164) dans le support (162).

14. Kit de traitement de la peau selon l'une quelconque des revendications 7 à 12, dans lequel l'applicateur (102) inclut des moyens de sécurité (180) configuré pour délivrer en sortie un signal de sécurité si la partie d'insertion (164) est correctement positionnée dans le support (162), dans lequel facultativement l'émetteur de micro-ondes (110) émet uniquement un rayonnement de micro-ondes lors de la réception du signal de sécurité.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19a

Fig. 19b

Fig. 19c

Fig. 20a

Fig. 20b

Fig. 21a

Fig. 21b

Fig. 21c

Fig. 21d

**EP 4 376 940 B1**

**Patent documents cited in the description**

- WO 2017202911 A1
- US 2015025513 A1
- US 10779885 B2
- US 2010296977 A1
- GB 2467604 A